(19) Europäisches Patentamt
European Patent Office
Office européen des brevets

(11) **EP 2 403 350 B1**

(12) **FASCICULE DE BREVET EUROPEEN**

(45) Date de publication et mention
de la délivrance du brevet:
**12.04.2017 Bulletin 2017/15**

(21) Numéro de dépôt: **10709910.3**

(22) Date de dépôt: **25.02.2010**

(51) Int Cl.:
*A23J 1/14* (2006.01)     *A23J 3/14* (2006.01)
*A23J 3/26* (2006.01)     *A23K 10/00* (2016.01)
*A23L 5/00* (2016.01)     *A23L 33/17* (2016.01)
*A23L 27/30* (2016.01)

(86) Numéro de dépôt international:
**PCT/FR2010/050328**

(87) Numéro de publication internationale:
**WO 2010/100369 (10.09.2010 Gazette 2010/36)**

(54) **POUDRE GRANULEE CONTENANT DES PROTEINES VEGETALES ET DES MALTODEXTRINES, LEUR PROCEDE D'OBTENTION ET LEURS UTILISATIONS**

GRANULAT MIT PFLANZENPROTEINEN UND MALTODEXTRINEN, HERSTELLUNGSVERFAHREN DAFÜR UND IHRE VERWENDUNG

GRANULATED POWDER CONTAINING VEGETABLE PROTEINS AND MALTODEXTRINS, METHOD FOR PRODUCING SAME, AND USES THEREOF

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **02.03.2009 FR 0951294**

(43) Date de publication de la demande:
**11.01.2012 Bulletin 2012/02**

(73) Titulaire: **Roquette Frères
62136 Lestrem (FR)**

(72) Inventeurs:
• **BOURSIER, Bernard
F-62138 Violaines (FR)**
• **PASSE, Damien
F-59500 Douai (FR)**

(74) Mandataire: **Gallois, Valérie et al
Cabinet BECKER & ASSOCIES
25, rue Louis Le Grand
75002 Paris (FR)**

(56) Documents cités:
**EP-A1- 0 796 567     EP-A2- 0 787 437
US-A- 6 056 949     US-A1- 2002 146 487**

## EP 2 403 350 B1

**Description**

<u>DOMAINE DE L'INVENTION</u>

La présente invention a pour objet une poudre granulée contenant des protéines végétales et des maltodextrines et/ou des sirops de glucose, ainsi que son procédé d'obtention et ses utilisations.

<u>ARRIERE-PLAN TECHNOLOGIQUE</u>

Les habitudes alimentaires se sont profondément transformées dans les pays industrialisés depuis la seconde guerre mondiale et même plus récemment sous l'impulsion de l'industrie agro-alimentaire dont l'influence croissante sur le comportement nutritionnel des populations tend à estomper progressivement les différences liées aux habitudes nutritionnelles traditionnelles. Cette évolution contribue vraisemblablement à accroître les risques lithiasiques, cardiovasculaires, diabétiques, d'obésité, de certains cancers d'origine nutritionnelle dans les sociétés industrielles où les besoins énergétiques quotidiens ont tendance à se réduire chez un nombre croissant d'individus ayant une activité de plus en plus sédentaire.

Les protéines représentent, après les glucides et les lipides, la troisième grande source énergétique de notre alimentation. Elles sont fournies aussi bien par des produits d'origine animale (viandes, poissons, oeufs, produits laitiers) que par des aliments végétaux (céréales, légumineuses,..). Les besoins quotidiens de protéines sont compris entre 12 et 20% de la ration alimentaire. Dans les pays industrialisés, ces apports sont majoritairement sous la forme de protéines d'origine animale. Les études le montrent, nous consommons trop de protéines d'origine animale (70% de nos apports en moyenne) et pas assez de protéines végétales (30%). De plus, notre alimentation est trop riche en lipides, notamment en acides gras saturés, en sucres, et trop pauvre en fibres. En matière d'apports protéiques, l'insuffisance comme l'excès sont préjudiciables : en cas d'apports insuffisants le développement et la croissance risquent d'être perturbés. En cas d'apports excessifs les acides aminés constitutifs des protéines sont oxydés ou convertis en glucides ou en graisses. Un tel excès n'est peut-être pas sans conséquence défavorable surtout dans le cas des protéines animales: outre le risque propre d'oxydation et de conversion des acides aminés, il faut se souvenir que les aliments riches en protéines animales le sont souvent aussi en lipides et en acides gras saturés. Une étude récente incrimine la responsabilité de l'excès de protéines animales dans la genèse d'une obésité ultérieure.

De plus, les atouts pour la santé sont évidents puisque la consommation excessive de protéines animales a été mise en avant dans les causes d'augmentation de certains cancers et maladies cardiovasculaires.

De plus, l'élevage intensif d'animaux génère de graves problèmes environnementaux. La production de viande requiert deux fois plus d'eau et de deux à quatre fois plus d'espace que la production nécessaire à une alimentation à base de plantes. L'élevage représente également une pollution importante des sols et de l'air. Il a été prouvé récemment que la pollution de l'élevage bovin dépassait la pollution automobile au niveau du rejet d'azote.

Enfin, l'élevage représente un formidable gâchis des ressources mondiales en eau : il faut 7 kg de céréales pour produire 1 kg de boeuf - 4 kg pour produire 1 kg de porc - 2 kg pour produire 1 kg de volaille. Les animaux d'élevage sont nourris avec des céréales comestibles par l'homme comme le soja (on parle alors de tourteau) et le maïs. Le soja est aujourd'hui au Brésil la principale cause de déforestation de l'Amazonie.

Ainsi les protéines animales issues de la viande présentent beaucoup de désavantages, tant sur le plan de la santé que sur le plan environnemental.

En parallèle, les protéines animales issues du lait ou des oeufs peuvent être allergènes, entraînant des réactions très gênantes, voire même dangereuses au quotidien.

Ainsi, les oeufs sont des trophallergènes (un type d'allergènes) qui pénètrent par voie digestive et qui provoquent chez certains individus une libération d'histamine par les cellules de l'organisme. C'est cette substance qui est responsable des symptômes de l'inflammation et qui entraîne la contraction des muscles des bronches. L'hypersensibilité est le plus souvent liée au blanc de l'oeuf. Par contre, chez certaines personnes ce sont les protéines contenues dans le jaune d'oeuf qui provoquent des réactions allergiques. L'allergie aux oeufs est particulière car elle cause la gamme complète des symptômes associés aux allergies alimentaires comme des ballonnements, des troubles digestifs, des éruptions cutanées, de la nausée, de la diarrhée, des crises d'asthme et de l'eczéma. L'allergie au blanc d'oeuf peut mener jusqu'au choc anaphylactique, une violente réaction pouvant entraîner la mort de la personne allergique si elle ne reçoit pas immédiatement une injection d'adrénaline.

L'allergie aux produits laitiers est une des réactions allergiques les plus répandues. Les études démontrent que 65 % des personnes qui souffrent d'allergies alimentaires sont allergiques au lait. La forme adulte de l'allergie au lait, appelée ici «allergie aux produits laitiers», est une réaction du système immunitaire qui crée des anticorps pour combattre l'aliment indésirable. Cette allergie est différente de l'allergie aux protéines du lait de vache (protéines bovines), qui touche les nouveau-nés et les enfants. L'allergie aux produits laitiers cause des symptômes variés tels que constipation, diarrhée, flatulences, eczéma, urticaire, nausées, migraines, infections, crampes abdominales, congestion nasale et même de graves crises d'asthme. Les personnes allergiques doivent complètement éliminer le lait, les produits laitiers et leurs dérivés de leur alimentation. Les termes suivants sont des indicateurs de présence de lait de vache ou de ses dérivés dans les ingrédients d'un produit : babeurre, caséinate de calcium, caséinate de sodium, caséine, caséinate, caséine hydrolysée, solides de lait séchés, lactalbumine, lactose, lactoglobuline, lait maigre, poudre de lait, lait condensé sucré et petit lait.

Un autre problème majeur lié aux protéines laitières est leur coût qui ne cesse d'augmenter. L'application des quotas laitiers a provoqué d'une part une réduction drastique de la quantité de protéines laitières disponibles pour la fabrication de produits alimentaires et d'autre part de fortes fluctuations de leurs prix. Les industriels cherchent de plus en plus des produits de substitution de ces protéines laitières.

Au vu de tous les désavantages, tant économiques, environnementaux que nutritionnels liés à la consommation de protéines animales issues de la viandes et/ou de produits dérivés, il en résulte un grand intérêt pour l'emploi de protéines de substitution, également appelées protéines alternatives, parmi lesquelles se classent les protéines végétales. Le marché alternatif de ces protéines se développe rapidement, pour de nombreuses raisons. Ces protéines ont une influence profonde sur la formulation des aliments équilibrés et des régimes basés sur un faible index glycémique (GI) et sur un fort apport protéique, et les fabricants traditionnels commencent à chercher de nouvelles sources de protéines pour enrichir leurs produits.

Par exemple, le document WO 2008/066308 décrit une composition alimentaire contenant une combinaison optimale de nutriments essentiels à une alimentation équilibrée associés à des protéines de soja. Cette composition permet de réduire les problèmes d'obésité en réduisant entre autre les apports protéiques néfastes.

Le document EP 0522800 décrit un nouveau procédé de traitement d'un concentré de protéine végétale permettant d'en améliorer sa fonctionnalité à lier la graisse et l'eau, ainsi que son application en remplacement des protéines animales dans la fabrication de saucisses.

Dans le document EP 0238946, il est décrit un isolat de protéines amélioré provenant de graines d'un légume à grains à teneur en lipides relativement basse, son procédé de préparation ainsi que son utilisation en tant qu'additif dans la fabrication de saucisses et cervelas.

US 6 056 949 décrit une poudre granulée aromatique comprenant une protéine végétale hydrolysée et de la maltodextrine.

EP 663 797 décrit dans l'exemple 1 une composition pulvérulente comprenant une protéine végétale et un hydrolysat d'amidon.

WO 98/19652 décrit une composition pulvérulente comprenant une matrice comprenant une protéine et un amidon hydrolysé.

WO 2005/063056 et WO 2005/063058 décrivent un procédé de préparation d'un précurseur pulvérulent permettant la préparation d'une boisson acide, ledit précurseur comprenant une protéine végétale et un hydrolysat d'amidon.

La société Demanderesse s'est également attelée à cette recherche afin de pouvoir répondre aux demandes croissantes des industriels pour des composés possédant des propriétés fonctionnelles intéressantes sans pour autant présenter les inconvénients de certains composés déjà existants.

En effet dans des domaines aussi diversifiés que l'alimentaire, la pharmacie, la cosmétique, l'agrochimie, les matériaux de construction, les papiers-cartons, les industriels sont constamment à la recherche de nouveaux composés possédant une image positive et bénéfique pour la santé, et susceptibles de modifier les propriétés fonctionnelles des milieux afin de fabriquer des produits possédant des textures variées.

C'est ainsi que la Demanderesse a réalisé d'importants travaux de recherche sur les Matières Protéiques Végétales (MPV) en tant qu'ingrédients alimentaires. Cet intérêt pour les MPV est tout d'abord dû à leurs nombreuses propriétés fonctionnelles, mais aussi à des qualités nutritionnelles intéressantes de par leur composition en acides aminés dits essentiels.

Par MPV on désigne dans la présente demande des ingrédients alimentaires obtenus à partir d'oléagineux, de légumineuses ou de céréales par réduction ou élimination de certains des principaux constituants non protéiques (eau, huile, amidon, autres glucides), de manière à obtenir une teneur protéique (N x 6,25) de 50% ou plus. La teneur protéique est calculée sur la base du poids sec à l'exclusion des vitamines et des sels minéraux.

Les MPV sont de plus en plus utilisées dans les applications alimentaires. Elles sont devenues un ingrédient important pour leurs propriétés foisonnantes, texturantes, émulsifiantes, épaississantes, stabilisantes, moussantes ou gélifiantes, sans cesse améliorées, pour une exploitation dans des applications connues ou encore tout simplement dans des créations totalement nouvelles.

Un des objets de la présente invention est donc de proposer des protéines végétales en remplacement des protéines animales, tout en permettant de conserver, au produit dans lequel elles sont utilisées, des propriétés fonctionnelles, une saveur et une palatabilité ainsi qu'une valeur nutritionnelle au moins similaires, voire améliorées. Le produit aura une valeur nutritionnelle équivalente :

- si sa qualité protéique n'est pas inférieure à celle du produit d'origine, et

- s'il renferme une quantité équivalente de protéines (N x 6,25) de sels minéraux et de vitamines à celle présente dans les produits d'origine animale.

Les protéines jouent un rôle majeur sur la qualité organoleptique de nombreux aliments frais ou manufacturés, comme par exemple la consistance et la texture de la viande et produits carnés, du lait et dérivés, des pâtes et du pain. Ces qualités des aliments dépendent très fréquemment de la structure et des propriétés physico-chimiques des composants protéiques ou tout simplement de leurs propriétés fonctionnelles.

Le terme propriétés fonctionnelles des ingrédients alimentaires signifie dans la présente demande toute propriété non nutritionnelle qui influence l'utilité d'un ingrédient dans un aliment. Ces diverses propriétés vont contribuer à l'obtention des caractéristiques finales désirées de l'aliment. Quelques unes de ces propriétés fonctionnelles sont la solubilité, l'hydratation, la viscosité, la coagulation, la stabilisation, la texturation, la formation de pâte, les propriétés moussantes et coagulantes.

Outre la substitution des protéines animales et de ce fait l'élimination de bien des désavantages liés à leur emploi, la Société Demanderesse s'est également concentrée sur la formulation de nouveaux ingrédients alimentaires prêts à l'emploi, contenant en plus des MPV d'autres composés présentant des propriétés fonctionnelles et/ou nutritionnelles différentes mais complémentaires.

En effet, de nos jours, dans un souci de rentabilité maximale, on assiste de plus en plus à une volonté de simplification des procédés de fabrication de la part des industriels, et tout particulièrement dans le secteur agroalimentaire.

Cette simplification des procédés de fabrication des produits alimentaires se traduit notamment par une réduction du nombre de composés utilisés, et notamment des ingrédients rentrant dans la préparation des produits finis. Cette réduction des ingrédients permet à la fois de limiter les temps de fabrication des produits, d'en simplifier les procédés et d'en réduire les coûts. Toutefois, elle ne doit pas en altérer la texture, ni les propriétés tant fonctionnelles, nutritionnelles, sensorielles qu'organoleptiques.

Toujours dans une volonté de simplifier les procédés de fabrication des produits alimentaires, les industriels émettent également de plus ne plus d'exigences quant à la forme desdits ingrédients utilisés. La forme sèche est de loin la forme préférée des industriels, tant sur le plan de la conservation, du stockage et de la manutention, par rapport à une forme liquide par exemple, beaucoup moins stable dans le temps. Et pourtant l'utilisation d'ingrédients sous forme pulvérulente présente le désavantage que ces produits présentent parfois des difficultés à se dissoudre, ce qui peut entraîner des phénomènes de décantation, une mauvaise dispersibilité avec formation de grumeaux et donc de répartition non uniforme des ingrédients au cours de procédé. Qui plus est, la manipulation de produits pulvérulents pose des problèmes de sécurité dus, entres autres, aux résidus secs que les manipulateurs peuvent respirer, avec également des risques d'incendies et d'explosion.

De tout ce qui précède, il résulte qu'il existe un réel besoin, non satisfait, de disposer d'une composition employée comme un substitut des protéines d'origine animale, possédant plusieurs propriétés fonctionnelles intéressantes lui permettant de réduire le nombre d'additifs employés dans la fabrication d'un produit fini tout en lui assurant des caractéristiques technologiques similaires à celles obtenues en employant séparément lesdits additifs, et se présentant sous une forme sèche mais non pulvérulente, pouvant être facilement hydratée.

Forte de ce constat et après de nombreux travaux de recherche, la Société Demanderesse a eu le mérite de concilier tous ces objectifs réputés jusqu'alors difficilement conciliables en proposant une nouvelle composition contenant entre autre des protéines végétales, caractérisée en ce qu'elle:

- associe une protéine végétale et un hydrolysat d'amidon, possédant lui-même, une caractéristique fonctionnelle, et/ou une caractéristique nutritionnelle, et/ou une caractéristique technologique intéressante et recherchée,
- se présente sous forme sèche mais non pulvérulente, c'est-à-dire sous forme granulaire, on parle de poudre granulée,
- présente une teneur en matière sèche supérieure à 80%, de préférence supérieure à 85%, et encore plus préférentiellement supérieure à 90%,
- présente un caractère « instant », c'est-à-dire que cette poudre granulée présente de très bonnes mouillabilité, dispersibilité et solubilité dans l'eau.

Ladite poudre granulée est caractérisée par le fait qu'elle présente, par rapport aux simples mélanges physiques de poudre décrits dans l'art antérieur, une meilleure dispersion dans l'eau et une meilleure dissolution à froid, une meilleure coulabilité pour les opérations de dosage, et qu'elle offre un meilleur environnement pour la manutention des poudres par l'absence de poussières. Qui plus est, cette poudre granulée présente des caractéristiques fonctionnelles améliorées, que le simple mélange physique des différents constituants n'aurait pas permis d'obtenir.

RESUME DE L'INVENTION

La présente invention a donc pour objet une poudre granulée comprenant au moins une protéine d'origine végétale et au moins un hydrolysat d'amidon, caractérisée en ce qu'elle présente un diamètre moyen volumique laser D4,3 compris entre 10$\mu$m et 500$\mu$m, de préférence entre 50$\mu$m et 350$\mu$m et encore plus préférentiellement entre 70$\mu$m et 250$\mu$m, et une matière sèche, déterminée après étuvage à 130°C pendant 2 heures, supérieure à 80%, de préférence supérieure à 85%, et encore plus préférentiellement supérieure à 90%,

dans laquelle ladite protéine végétale est une protéine de pois, ledit hydrolysat d'amidon est une maltodextrine dont le DE est compris entre 15 et 19 ,

dans laquelle la somme des quantités de protéine végétale et d'hydrolysat d'amidon est comprise entre 50 et 100% de la masse totale de ladite poudre granulée (sec/sec),

dans laquelle le ratio pondéral de la protéine végétale à l'hydrolysat d'amidon est compris entre 80 :20 et 45 :55 , et dans laquelle les protéines végétales présentent plus de 50 % de protéines de plus de 1.000 Da.

La présente invention concerne également le procédé d'obtention de cette poudre granulée ainsi que son utilisation dans différents secteurs industriels, et plus particulièrement dans le secteur agroalimentaire où elle est utilisée en tant qu'agent fonctionnel tel qu'agent émulsifiant, foisonnant, stabilisant, épaississant et/ou gélifiant, notamment en substitution de tout ou partie de certaines protéines animales dans la préparation de produits alimentaires.

DESCRIPTION DETAILLEE DES MODES DE REALISATION

La présente invention est relative à une poudre granulée comprenant au moins une protéine végétale et au moins un hydrolysat d'amidon, caractérisée en ce qu'elle présente un diamètre moyen volumique laser D4,3 compris entre 10$\mu$m et 500$\mu$m, de préférence entre 50$\mu$m et 350$\mu$m et encore plus préférentiellement entre 70$\mu$m et 250$\mu$m, et une matière sèche, déterminée après étuvage à 130°C pendant 2 heures, supérieure à 80%, de préférence supérieure à 85%, et encore plus préférentiellement supérieure à 90%,

dans laquelle ladite protéine végétale est une protéine de pois, ledit hydrolysat d'amidon est une maltodextrine dont le DE est compris entre 15 et 19 ,

dans laquelle la somme des quantités de protéine végétale et d'hydrolysat d'amidon est comprise entre 50 et 100% de la masse totale de ladite poudre granulée (sec/sec),

dans laquelle le ratio pondéral de la protéine végétale à l'hydrolysat d'amidon est compris entre 80 :20 et 45 :55 , et dans laquelle les protéines végétales présentent plus de 50 % de protéines de plus de 1.000 Da.

Dans la présente invention, ladite poudre granulée est caractérisée en ce que le ratio pondéral de la protéine végétale à l'hydrolysat d'amidon est compris entre 80 :20 et 45 :55, plus préférentiellement encore entre 65 :35 et 45 :55et en particulier entre 55 :45 et 45 :55.

Par « légumineuses » au sens de la présente invention, on entend toutes plantes appartenant aux familles des césalpiniacées, des mimosacées ou des papilionacées et notamment toutes plantes appartenant à la famille des papilionacées comme, par exemple, le pois, le haricot, la fève, la féverole, la lentille, la luzerne, le trèfle ou le lupin.

Cette définition inclut notamment toutes les plantes décrites dans l'un quelconque des tableaux contenus dans l'article de R. HOOVER et al., 1991 (HOOVER R. (1991) « Composition, structure, functionality and chemical modification of legume starches : a review » Can. J. Physiol. Pharmacol., 69 pp. 79-92).

Ladite protéine de légumineuse est le pois.

Le terme « pois » étant ici considéré dans son acception la plus large et incluant en particulier :

- toutes les variétés sauvages de « pois lisse » (« smooth pea ») et de « pois ridés » (« wrinkled pea »), et
- toutes les variétés mutantes de « pois lisse » et de « pois ridé » et ce, quelles que soient les utilisations auxquelles on destine généralement lesdites variétés (alimentation humaine, nutrition animale et/ou autres utilisations).

Lesdites variétés mutantes sont notamment celles dénommées « mutants r », « mutants rb », « mutants rug 3 », « mutants rug 4 », « mutants rug 5 » et « mutants lam » tels que décrits dans l'article de C-L HEYDLEY et al. intitulé « Developing novel pea starches » Proceedings of the Symposium of the Industrial Biochemistry and Biotechnology Group of the Biochemical Society, 1996, pp. 77-87.

De manière encore plus préférentielle, ladite protéine de légumineuse est le pois lisse.

En effet, le pois est la légumineuse à graines riches en protéines qui, depuis les années 70, s'est le plus développée en Europe et principalement en France, non seulement comme source protéique pour l'alimentation animale, mais aussi pour l'alimentation humaine.

Les protéines de pois sont constituées, comme toutes les protéines de légumineuses, de trois classes de protéines principales: les globulines, les albumines et les protéines dites « insolubles ».

L'intérêt des protéines de pois réside dans leurs bonnes capacités émulsifiantes, leur absence d'allergènicité, et leur faible coût, ce qui en fait un ingrédient fonctionnel économique.

De plus, les protéines de pois participent favorablement au développement durable et leur impact carbone est très positif. En effet, la culture du pois est respectueuse de l'environnement, et ne nécessite pas d'engrais azotés, car le pois fixe l'azote de l'air.

En outre, sous forme native globulaire, les protéines de pois sont solubles dans l'eau, ce qui permet d'envisager de les incorporer dans des émulsions.

Par protéine de pois, on désigne, de manière préférée selon la présente invention, les protéines de pois qui sont principalement sous forme native, globulaire, globulines, ou albumines.

De manière encore plus préférentielle, les protéines végétales, et en particulier les protéines de pois, utilisées selon l'invention sont sous forme d'une composition de protéine végétale, et en particulier de protéine de pois, présentant :

- une teneur en protéines totales (N x 6,25), exprimée en grammes de produit sec, d'au moins 60% en poids de produit sec. De préférence, on utilise dans le cadre de la présente invention une composition de protéine ayant une teneur en protéines élevée comprise entre 70% et 97% en poids de produit sec, de préférence comprise entre 76% et 95%, plus préférentiellement encore comprise entre 78% et 88%, et en particulier comprise entre 78% en 85%,
- une teneur en protéines solubles, exprimée selon un test de mesure de la solubilité dans l'eau des protéines, comprise entre 20 et 99%. De préférence, on utilise dans le cadre de la présente invention une composition de protéines ayant un taux élevé de protéines solubles compris entre 35 et 95%, de préférence entre 45 et 90%, plus préférentiellement encore entre 50 et 80%, et en particulier entre 55 et 75%.

Pour déterminer le taux de protéines totales, on peut effectuer le dosage de la fraction azotée soluble contenue dans l'échantillon selon la méthode de Kjeldahl, puis on obtient le taux de protéines totales en multipliant le taux d'azote exprimé en pourcentage de poids de produit sec par le facteur 6,25. Cette méthode est bien connue de l'homme du métier.

Dans la présente invention, le taux de protéines totales peut également être mesuré par le dosage de la fraction azotée soluble contenue dans l'échantillon selon la méthode de Dumas A., 1831, Annales de chimie, 33, 342, comme cité par Buckee, 1994, dans Journal of the Institute of Brewing, 100, pp 57-64, puis on obtient le taux de protéines totales en multipliant le taux d'azote exprimé en pourcentage de poids de produit sec par le facteur 6,25. Cette méthode, également connue comme méthode de dosage de l'azote par combustion, consiste en une combustion totale de la matrice organique sous oxygène. Les gaz produits sont réduits par du cuivre puis desséchés et le gaz carbonique est piégé. L'azote est ensuite quantifié à l'aide d'un détecteur universel. Cette méthode est bien connue de l'homme du métier.

Pour déterminer le taux de protéines solubles, on mesure la teneur en protéines solubles dans l'eau dont le pH est ajusté à 7,5 +/- 0,1 à l'aide d'une solution de HCl ou NaOH, par une méthode de dispersion d'une prise d'essai de l'échantillon dans de l'eau distillée, centrifugation et analyse du surnageant. Dans un bécher de 400 ml, on introduit 200,0 g d'eau distillée à 20°C +/- 2°C, et on place le tout sous agitation magnétique (barreau aimanté et rotation à 200 rpm). On ajoute exactement 5 g de l'échantillon à analyser. On agite pendant 30 min, et on centrifuge pendant 15 min à 4.000 rpm. On réalise, sur le surnageant la méthode de détermination de l'azote selon la méthode précédemment décrite.

Ces compositions de protéines végétales, et en particulier de protéines de pois, présentent de manière préférée plus de 60, 70, 80 ou 90 % de protéines de plus de 1.000 Da. En outre, ces compositions de protéines végétales, et en particulier de protéines de pois, présentent de manière préférée un profil de distribution des poids moléculaires constitué de :

- 1 à 8%, de préférence de 1,5 à 4%, et plus préférentiellement encore de 1,5 à 3% de protéines de plus de 100.000 Da,
- 20 à 55%, de préférence de 25 à 55% de protéines de plus de 15.000 et d'au plus de 100.000 Da,
- 15 à 30% de protéines de plus de 5.000 et d'au plus de 15.000 Da,
- et de 25 à 55%, de préférence de 25 à 50%, et plus préférentiellement encore de 25 à 45% de protéines d'au plus de 5.000 Da.

La détermination des poids moléculaires des protéines constitutives desdites compositions de protéines végétales, et en particulier de pois est réalisée par chromatographie d'exclusion stérique en conditions dénaturantes (SDS + 2-mercaptoéthanol); la séparation se fait en fonction de la taille des molécules à séparer, les molécules de taille élevée étant éluées en premier.

Des exemples de compositions de protéines de pois selon l'invention, ainsi que le détail de la méthode de détermination des poids moléculaires peuvent être trouvés dans le brevet WO 2007/017572 dont la Société Demanderesse est également titulaire.

Selon la présente invention, les dites protéines végétales, et en particulier de pois, utilisées pour l'obtention de la poudre granulée peuvent également être des « concentrats de protéines végétales » ou des « isolats de protéines végétales », de préférence des « concentrats de protéines de pois » ou des « isolats de protéines de pois ». Les concentrats et les isolats de protéines végétales, et en particulier de pois, sont définis en regard de leur contenu en protéines (cf. la revue de J. GUEGUEN de 1983 dans Proceedings of european congress on plant proteins for human food (3-4) pp 267 - 304) :

- les concentrats de protéines végétales, et en particulier de pois, sont décrits comme présentant un contenu en protéines totales de 60 à 75 % sur sec, et
- les isolats de protéines végétales, et en particulier de pois, sont décrits comme présentant un contenu en protéines totales de 90 à 95 % sur sec,

les teneurs en protéines étant mesurées par la méthode de Kjeldhal (cf. ci-avant), la teneur en azote étant multiplié par le facteur 6,25.

La poudre granulée comprend au moins une protéine végétale et au moins un hydrolysat d'amidon.

Dans la présente invention, le terme « hydrolysat d'amidon » désigne tout produit obtenu par hydrolyse acide ou enzymatique d'amidon de légumineuses, de céréales ou de tubercules. Divers procédés d'hydrolyse sont connus et ont été décrits de manière générale aux pages 511 et 512 de l'ouvrage Encyclopedia of Chemical Technology de Kirk-Othmer, 3ème Edition, Vol. 22, 1978. Ces produits d'hydrolyse se définissent également comme des mélanges purifiés et concentrés formés de chaînes linéaires constituées d'unités D-glucose et de polymères de D-glucose essentiellement lié en $\alpha(1{\rightarrow}4)$ avec seulement de 4 à 5 % de liaisons glucosidiques ramifiées $\alpha(1{\rightarrow}6)$, de poids moléculaires extrêmement variés, complètement solubles dans l'eau. Les hydrolysats d'amidon sont très bien connus et parfaitement décrits dans Encyclopedia of Chemical Technology de Kirk-Othmer, 3ème Edition, Vol. 22, 1978, pp. 499 à 521.

Ainsi, dans la présente invention, le produit d'hydrolyse de l'amidon est choisi parmi les maltodextrines.

La distinction entre les produits d'hydrolyse de l'amidon repose principalement sur la mesure de leur pouvoir réducteur, exprimé classiquement par la notion de Dextrose Equivalent ou DE. Le DE correspond à la quantité de sucres réducteurs, exprimée en équivalent dextrose pour 100g de matière sèche du produit. Le DE mesure donc l'intensité de l'hydrolyse de l'amidon, puisque plus le produit est hydrolysé, plus il contient de petites molécules (telles que le dextrose et le maltose par exemple) et plus son DE est élevé. Au contraire, plus le produit contient de grandes molécules (polysaccharides), plus son DE est bas.

Du point de vue réglementaire, les maltodextrines ont un DE compris de 1 à 20, et les sirops de glucose ont un DE supérieur à 20.

Selon la présente invention, la poudre granulée comprend une protéine de pois et une maltodextrine possédant un DE compris entre 15 et 19.

Selon une variante de l'invention, la poudre granulée comprend une protéine de pois et un mélange de maltodextrines et de sirop de glucose.

Selon un mode avantageux de cette variante, la poudre granulée comprend une protéine de pois et un mélange de maltodextrine possédant un DE compris entre 15 et 19 et de sirop de glucose dont le DE n'excède pas la valeur de 47, et de préférence 35.

Dans le cadre de la présente invention, l'expression « poudre granulée » signifie qu'il existe un mélange intime entre les différents composants de cette poudre, que leur répartition au sein de la poudre est sensiblement homogène, et que ceux-ci ne sont pas uniquement liés entre eux par un simple mélange physique. Des interactions entre les constituants peuvent survenir, aussi bien à l'extérieur de la particule qu'à l'intérieur.

Dans un mode de réalisation particulier, la poudre granulée n'est pas enrobée.

Par opposition, dans la présente invention, l'expression « simple mélange » signifie qu'il n'existe pas de mélange intime entre les différents constituants, et qu'il n'y a eu qu'un simple mélange physique par contact. Il n'y a aucune interaction entre les constituants car ils ne sont quasiment pas en contact entre eux.

En effet, pour obtenir ladite poudre granulée, la Société Demanderesse a constaté qu'il convenait d'employer un mélange d'au moins une protéine végétale et d'au moins un hydrolysat d'amidon, et de modifier ses caractéristiques physiques en employant un procédé approprié, de telle sorte que l'on obtienne simultanément des propriétés fonctionnelles très intéressantes ne pouvant être obtenues si chaque composé est utilisé séparément ou si les composés sont utilisés simultanément mais sous la forme d'un simple mélange de poudres.

Dans la présente invention, ladite poudre granulée est préparée par un procédé de séchage selon une technique choisie dans le groupe constitué par l'atomisation, la granulation, l'extrusion ou par tout autre moyen de séchage connu de l'homme du métier, et dans des conditions adaptées à l'équipement choisi, susceptible de permettre l'obtention d'une poudre granulée selon l'invention.

Ainsi, la présente invention vise également un procédé de fabrication de la susdite poudre granulée. Ledit procédé de fabrication consiste à sécher conjointement au moins deux constituants, et comprend une étape de mise en contact intime d'au moins une protéine végétale avec au moins un hydrolysat d'amidon, ces étape de mise en contact intime pouvant être menées selon toute méthode connue de l'homme du métier, et notamment selon une technique choisie parmi l'atomisation, la granulation et l'extrusion, et toute combinaison d'au moins deux de ces techniques, telle que ladite étape de mise en contact intime conduise à une matière sèche déterminée après étuvage à 130°C pendant 2 heures, supérieure à 80%, de préférence supérieure à 85%, et encore plus préférentiellement supérieure à 90%, dans lequel ladite protéine végétale est une protéine de pois, ledit hydrolysat d'amidon est une maltodextrine dont le DE est compris entre 15 et 19, et le ratio pondéral de la protéine végétale à l'hydrolysat d'amidon est compris entre 80 :20 et 45 :55. A titre d'exemple, on citera un procédé de fabrication de ladite poudre granulée selon une unique technique d'atomisation, ou selon une unique technique de granulation, ou encore selon une combinaison entre une technique d'atomisation suivie d'une technique de granulation.

Ainsi, selon une première variante de l'invention, ladite poudre granulée est susceptible d'être obtenue selon un procédé de fabrication qui comprend une étape d'atomisation d'une suspension d'au moins une protéine végétale et d'au moins un hydrolysat d'amidon, ladite étape d'atomisation étant suivie par une étape de granulation de la poudre « atomisée » sur un granulateur. Selon cette première variante, une suspension à atomiser est préparée, contenant au moins une protéine de pois, et au moins une maltodextrine de DE compris entre 15 et 19, dans les proportions requises. Toujours selon cette variante, on peut également envisager de préparer une suspension aqueuse à atomiser par constituant.

Toujours selon cette variante, la suspension à atomiser peut être préparé soit à partir d'une composition sèche de protéines de pois, i.e. sous la forme d'une poudre qui est ensuite diluée dans de l'eau, soit à partir d'un floc de protéines de pois. Dans cette deuxième alternative, le floc de protéines de pois est obtenu après broyage de la farine de pois, remise de cette farine broyée en suspension dans l'eau, puis fractionnement de ladite suspension par tout moyen connu par ailleurs de l'homme du métier, de manière à isoler une fraction riche en protéines. Puis, les protéines sont isolées de cette fraction par une technique choisie dans le groupe des techniques de précipitation des protéines à leur pH isoélectrique et des techniques de séparation membranaire de type ultrafiltration. Enfin, la séparation du précipité (encore appelé " floc ") renfermant les protéines solubles s'effectue sur décanteur centrifuge ou en séparatrice à assiettes. Le floc peut être utilisé tel quel ou mis en suspension, selon sa matière sèche.

L'étape d'atomisation est une opération unitaire de séchage qui consiste à transformer en poudre un liquide, pulvérisé sous forme de gouttelettes mises en contact avec un gaz chaud. Cette opération détermine la taille des gouttelettes produites (et leur granulométrie), leur trajectoire, leur vitesse et par conséquent la dimension finale des particules sèches, ainsi que les propriétés des poudres obtenues : écoulement, caractère instant lié à leur solubilité, densité, comprimabilité, friabilité,...

L'étape d'atomisation peut être réalisée dans un atomiseur ou une tour d'atomisation, dans laquelle ladite suspension (ou les suspensions) à sécher est divisée dans un courant de gaz chaud qui apporte les calories nécessaires à l'évaporation du solvant et absorbe, pour l'évacuer, l'humidité libérée par le produit en cours de séchage. Le mélange liquide est admis au sommet par une buse ou une turbine, la poudre « atomisée » obtenue est récoltée à la base de la tour. Le solide sec est séparé du gaz d'atomisation par un (ou des) cyclone(s), ou par filtration (filtre à manches par exemple). Dans certains cas, si cela s'avère nécessaire, la tour peut être remplie d'un gaz inerte pour éviter les phénomènes d'oxydation.

L'étape de granulation a lieu après l'étape d'atomisation, et consiste à la pulvérisation d'une solution aqueuse sur la poudre issue de l'étape d'atomisation. Une telle opération, réunissant une étape d'atomisation suivie d'une étape de granulation, est classiquement mise en oeuvre dans un atomiseur multiples effets comme par exemple une Tour MSD (pour Multi-Stage Dryer).

Selon un mode de réalisation préférentiel de cette première variante, on peut procéder selon les étapes suivantes :

1) préparer à une température comprise entre 15 et 70°C, et de préférence entre 15 et 50°C une suspension de protéines de pois et d'hydrolysats d'amidon, dans laquelle :

- lesdites protéines de pois possèdent une teneur en protéines solubles comprise entre 20 et 99%, de préférence entre 45 et 90%, plus préférentiellement encore entre 50 et 80%, et en particulier entre 55 et 75% ;
- lesdits hydrolysats d'amidon étant choisis dans le groupe constitué par les maltodextrines dont le DE est compris entre 15 et 19;
- le ratio pondéral des protéines de pois aux hydrolysats d'amidon est compris entre 80 :20 et 45 :55 , plus préférentiellement encore entre 65 :35 et 45 :55 et en particulier entre 55 :45 et 45 :55 ;
- la matière sèche de la suspension est comprise entre 25 et 50%, de préférence entre 30 et 40%,

1') réaliser une première étape optionnelle de traitement thermique à température élevée et pendant un temps court afin de réduire les risques bactériologiques de la suspension obtenue selon 1, ledit traitement pouvant être choisi parmi les traitements HTST (High Temperature Short Time), UHT ;
1") réaliser une seconde étape optionnelle, d'homogénéisation à haute pression de la suspension obtenue selon 1), et indépendamment de la première étape optionnelle ;

2) maintenir ou ramener en cas de réalisation de l'étape 1'), ladite suspension de protéines de pois et d'hydrolysats d'amidon à une température comprise entre 15 et 80°C, et de préférence entre 15 et 50°C ;

3) atomiser ladite suspension dans une tour d'atomisation de type MSD équipée d'une buse d'atomisation haute pression avec recyclage des fines particules en tête de tour ;

4) granuler dans ladite tour d'atomisation ;

5) récupérer la poudre granulée ainsi obtenue et comprenant les protéines de pois et les hydrolysats d'amidon.

Comme il sera exemplifié ci-après, la société Demanderesse recommande d'utiliser une tour de type MSD 20 commercialisée par la société NIRO.

La buse d'injection est choisie de manière à obtenir une pression comprise entre 50 et 300 bars, de préférence de l'ordre de 150 bars, pour un débit compris entre 100 et 150 1/h, de préférence de l'ordre de 120 1/h.

La température des airs d'entrée sont réglées de la manière suivante :

- pour l'air d'entrée en amont de la tête de tour : température comprise entre 150 et 180°C, de préférence 155°C,
- pour le lit fluidisé statique : température comprise entre 50 et 120°C, de préférence 84°C,
- pour le lit fluidisé vibré : température de l'ordre de 20°C.

La température de sortie est alors comprise entre 55 et 80°C, de l'ordre de 60°C.

La poudre granulée selon l'invention, contenant des cogranulés, est enfin récupérée en sortie de tour d'atomisation.

Selon une seconde variante de l'invention, ladite poudre granulée est obtenue selon un unique procédé de granulation qui permet de réaliser l'étape de mise en contact intime entre les différents constituants. Le procédé de granulation peut faire appel à deux techniques bien connues par l'homme du métier : la technique de granulation par voie sèche et la technique de granulation par voie humide.

Selon un mode préférentiel de cette seconde variante, la poudre granulée est obtenue par granulation par voie humide dans un lit fluidisé. Un exemple d'une telle granulation est par exemple cité dans le brevet EP 1 558 094 dont la demanderesse est titulaire.

Selon une troisième variante de l'invention, ladite poudre granulée est obtenue selon un unique procédé d'extrusion. Dans ce procédé, on utilisera une installation comportant au moins une filière d'extrusion, les paramètres de températures étant sélectionnés aisément par l'homme du métier en fonction de la teneur en eau de la composition avant séchage. La composition extrudée est ensuite successivement soumise à un refroidissement, à un broyage, et éventuellement un tamisage pour conduire à la poudre atomisée selon la présente invention.

Par la mise en oeuvre des procédés de séchage décrits précédemment, ou par tout autre moyen de séchage connu de l'homme du métier et dans des conditions adaptées à l'équipement choisi, on obtient une poudre granulée composée de cogranulés et contenant les différents composés de départ liés entre eux de façon intime.

La taille moyenne de la poudre obtenue conformément à l'invention peut être caractérisée par son diamètre moyen volumique (moyenne arithmétique) D 4,3. Il est compris entre $10\mu m$ et $500\mu m$, de préférence entre $50\mu m$ et $350\mu m$ et encore plus préférentiellement entre $70\mu m$ et $250\mu m$. Selon un mode préférentiel, le diamètre moyen volumique D 4,3 de la dite poudre granulée est compris entre $150\mu m$ et $240\mu m$.

Ces valeurs sont déterminées sur un granulomètre à diffraction LASER type LS 230 de la société BECKMAN-COULTER, équipé de son module de dispersion poudre (voie sèche), en suivant le manuel technique et les spécifications du constructeur. La gamme de mesure du granulomètre à diffraction LASER type LS 230 est de $0,04\ \mu m$ à $2.000\ \mu m$.

Selon un mode particulier de la présente invention, 90% de la poudre a un diamètre inférieure à $1000\mu m$, de préférence inférieur à $500\mu m$, et plus préférentiellement encore inférieur à $400\mu m$. En particulier, 90% de la poudre a un diamètre inférieur à $370\mu m$. Cette valeur correspond au $d_{90}$.

Selon un autre mode particulier de la présente invention, 50% de la poudre a un diamètre inférieur à $500\mu m$, de préférence inférieur à $300\mu m$, et plus préférentiellement encore inférieur à $250\mu m$. En particulier, 50% de la poudre a un diamètre inférieur à $220\mu m$. Cette valeur correspond au $d_{50}$.

Selon un autre mode particulier de la présente invention, 10% de la poudre a un diamètre inférieur à $300\mu m$, de préférence inférieur à $200\mu m$, et plus préférentiellement encore inférieur à $150\mu m$. En particulier, 10% de la poudre a un diamètre inférieur à $100\mu m$. Cette valeur correspond au $d_{10}$.

Ces trois valeurs $d_{90}$, $d_{50}$ et $d_{10}$ sont également déterminées à l'aide du granulomètre à diffraction LASER utilisé pour la détermination du diamètre moyen volumique D 4,3.

Selon un mode préférentiel de la présente invention, la poudre granulée contient des protéines de pois associées à des maltodextrines dont le DE est compris entre 15 et 19.

Selon l'invention, la poudre granulée contient des proportions de protéines végétales et d'hydrolysats d'amidon variables.

Selon un mode préférentiel, le ratio pondéral de la protéine de pois à l'hydrolysat est compris entre 75 :25 et 45 :55, plus préférentiellement entre 65 :35 et 45 :55. En particulier, ledit ratio est compris entre 55 :45 et 45 :55.

Ainsi, selon la présente invention, deux paramètres sont à considérer dans la matrice protéine végétale/hydrolysat d'amidon. Tout d'abord le premier paramètre variable est le ratio de chaque constituant par rapport à l'autre et le second est le DE de l'hydrolysat d'amidon employé. Ainsi pour un ratio identique, plusieurs compositions de poudre granulée peuvent être obtenues selon la présente invention, en fonction du DE de l'hydrolysat d'amidon employé.

Selon un autre mode préférentiel, la somme des quantités de protéines végétales, et de préférence de protéines de pois, et d'hydrolysats d'amidon est comprise entre 50 et 100%, de la masse totale de ladite poudre granulée (sec/sec).

La Société Demanderesse a eu le mérite de découvrir qu'en fonction de ces ratios, les propriétés fonctionnelles de la poudre pouvaient être différentes.

Dans un mode de réalisation selon l'invention, il a été observé de manière inattendue que dans le domaine alimentaire par exemple, la poudre granulée selon la présente invention présente l'avantage complémentaire de se substituer totalement ou partiellement aux matières grasses couramment utilisées dans les recettes.

Selon un autre mode de réalisation de l'invention, la poudre granulée comprend des protéines de pois et des hydrolysats d'amidon, et peut de plus contenir tout additif approprié, comme des arômes, des colorants, des agents stabilisants, des excipients, des lubrifiants, des conservateurs, dès lors qu'ils n'impactent pas de façon négative les propriétés fonctionnelles finales recherchées,

Il peut également s'agir de principes actifs pharmaceutiques ou phytosanitaires, de détergents. On entend par principe actif, dans la présente invention, toute molécule active possédant un effet pharmacologique démontré et un intérêt thérapeutique également démontré cliniquement.

Ladite poudre granulée conforme à l'invention peut, en outre, être également caractérisée par sa masse volumique apparente, déterminée selon la méthode de mesure préconisée par la Pharmacopée Européenne (PE 5.1 tome 1, 01/2005 : 20915 paragraphe 2-9-15 ; équipement selon la figure 2-9-15-1).

Dans ces conditions, ladite poudre granulée présente avantageusement une masse volumique apparente comprise entre 0,30 et 0,90 g/ml, de préférence comprise entre 0,40 et 0,60 g/ml.

Une autre propriété fonctionnelle de la poudre granulée conforme à l'invention est de posséder une excellente mouillabilité, bien meilleure que la mouillabilité mesurée pour le simple mélange. Cette caractéristique est la capacité d'absorption d'eau à la surface d'une poudre. Elle est proportionnelle à la solubilité de la poudre et inversement proportionnelle à la formation de grumeaux. Une grande mouillabilité permet de conférer le caractère dit « instant » à la poudre granulée de la présente invention.

Pour mesurer cette mouillabilité, on utilise un bécher haut de contenance 500 ml dans lequel on introduit 250 g d'eau distillée à 20°C +/- 2°C. On pèse exactement 25 g d'une poudre granulée conforme à l'invention ou 25 g du simple mélange. A t=0h on introduit rapidement et en une seule fois les 25 g d'échantillon et on déclenche le chronomètre. On mesure le temps nécessaire pour que l'échantillon se mouille complètement, c'est-à-dire pour qu'il ne reste plus d'échantillon sous forme sèche. Le test est réalisé sans agitation et sous agitation douce à 250 rpm. Dans le test sans agitation, la poudre granulée conforme à la présente invention se mouille en moins d'une minute, de préférence en moins de 30 secondes et plus préférentiellement encore en moins de 10 secondes, alors que le simple mélange met plus de 10 minutes pour se mouiller totalement.

Dans le test sous agitation douce, ladite poudre granulée se mouille en moins de 30 s, de préférence en moins de 10 s et plus préférentiellement encore en moins de 4s, alors que le simple mélange met plus de 3 minutes pour se mouiller totalement.

En particulier, et à titre d'exemple, selon le test de mouillabilité sans agitation décrit ci-dessus, une poudre granulée composée de protéines de pois et de maltodextrines de DE 19 se mouille en moins de 10 secondes, très exactement en 7 secondes alors que le simple mélange met 3min10s pour se mouiller totalement.

Ce test permet de démontrer que la poudre granulée possède un caractère dit « instant » en comparaison du simple mélange qui lui n'en possède pas.

La poudre granulée de la présente invention possède également une absence totale de décantation, c'est-à-dire une excellente tenue en suspension, ce qui facilite grandement sa mise en oeuvre dans les procédés industriels, et représente un avantage majeur.

La tenue en suspension est mesurée dans une éprouvette graduée de 250 ml. Après reconstitution d'une solution de 250 ml à 15% de poudre granulée selon l'invention, notamment par remise en suspension de ladite poudre par une agitation douce, le volume décanté est mesuré toutes les heures pendant 7 heures, puis au bout de 24h et de 48h. Il n'y a aucune décantation de la poudre granulée, et ce même après 48h d'attente. Cette absence totale de décantation ne se retrouve pas avec le simple mélange. En effet, une heure après la reconstitution du mélange on observe un phénomène de décantation qui s'accentue avec le temps.

D'autres propriétés technologiques très intéressantes conférées par ladite poudre granulée concernent ses pouvoirs émulsifiant, moussant et gélifiant, en comparaison au simple mélange des constituants de cette poudre.

Les propriétés émulsifiantes sont dues à la faculté de réduire les tensions interfaciales entre composants hydrophiles et hydrophobes d'un aliment. Elles sont directement liées à la solubilité de la protéine. Les poudres possédant ces propriétés de surface auront un potentiel d'utilisation important dans les émulsions en général, dans les poudres de lait, réengraissées ou non, ainsi que dans les aliments contenant eau et graisses (charcuterie, viande, condiment).

Dans la présente invention, la capacité émulsifiante correspond au pourcentage de « crème d'émulsion », formée et stable après centrifugation, en fonction de la quantité en protéines et de la quantité d'huile. Pour la mesurer, on prépare, sur un ultraturax à 9500 rpm pendant 1 minute, une émulsion de 50% d'huile de colza par une solution de poudre granulée (hydratées 10 minutes dans de l'eau déminéralisée afin de s'affranchir des forces ioniques) à 2%. Puis l'émulsion est centrifugée pendant 5 minutes à 1500g. Le volume de crème est mesuré en ml. La capacité émulsifiante (CE) se calcule par la formule suivante :

```
CE (en %) = (volume de crème / volume total) x 100
```

La poudre granulée présente une capacité émulsifiante supérieure à 50%, de préférence supérieure à 55% et encore plus préférentiellement supérieure à 60%, alors que les simples mélanges ont une capacité émulsifiante réduite, inférieure à 20%.

En particulier, et à titre d'exemple, selon le test de mesure de la CE décrit ci-dessus, une poudre granulée comprenant des protéines de pois et des maltodextrines de DE 19 présente une CE de 87,5%.

Les propriétés moussantes, qui sont très appréciées en pâtisseries (cakes, soufflets, meringues) et dans la fabrication de mousses, laitières ou autres, de crèmes fouettées, résultent d'un déplissement partiel des protéines qui s'orientent à l'interface eau/air.

Dans la présente invention, le pouvoir moussant est mesuré dans une éprouvette graduée de 500 ml. Une solution à 15% de poudre granulée conforme à la présente invention est préparée sur un ultraturax à 9500 rpm pendant 1 minute, avant d'être transférée dans l'éprouvette graduée. Les volumes de mousse et de liquide sont mesurés toutes les 10 minutes pendant 30 minutes. Le temps nécessaire pour que la mousse atteigne 50% de son volume initial est également mesuré et permettra de quantifier la stabilité de la mousse.

La poudre granulée possède un excellent pouvoir moussant, extrêmement stable en fonction du temps alors que le simple mélange ne mousse que très peu, et d'une mousse qui est instable au cours du temps.

Ainsi la poudre granulée possède des propriétés fonctionnelles (pouvoir émulsifiant, capacité moussante) qui lui ont été conférées notamment par son procédé de préparation.

Une autre propriété très intéressante conférée par la dite poudre granulée selon la présente invention est la nette amélioration du goût d'une part, de la palatabilité et du corps défini également par la viscosité en bouche d'autre part. En effet, la poudre granulée possède un goût neutre, contrairement au simple mélange qui peut présenter un goût de légumineuse plus marqué et être par conséquent un frein à certaines applications alimentaires. Dans certaines applications, la palatabilité et le corps se trouvent également améliorés par rapport au simple mélange.

Ces propriétés fonctionnelles très intéressantes et n'existant pas sur de simple mélange les destinent entre autre à des applications très diversifiées et variées.

Un autre aspect de la présente invention concerne l'utilisation de la poudre granulée dans les domaines de la cosmétique, de la détergence, de l'agrochimie, des formulations industrielles, pharmaceutiques, des matériaux de construction, des fluides de forage, en fermentation, en nutrition animale et dans des applications alimentaires.

Par conséquent, la présente invention concerne également des compositions cosmétique, détergente, agrochimique, des formulations industrielles, pharmaceutiques, des matériaux de construction, des fluides de forage, des milieux de fermentation, des compositions nutritionnelles animales, des applications alimentaires comprenant la poudre granulée selon la présente invention ou susceptible d'être obtenue selon la mise en oeuvre du procédé de préparation de poudre granulé selon l'invention tel que décrit ci-dessus.

Dans ces domaines, la poudre granulée selon l'invention peut être utilisée dans des compositions en tant qu'agent fonctionnel tel qu'agent émulsifiant, foisonnant, stabilisant, épaississant et/ou gélifiant, notamment en substitution de tout ou partie des protéines animales.

Par conséquent, la présente invention concerne également un agent émulsifiant, foisonnant, stabilisant, épaississant et/ou gélifiant, pouvant être utilisé en substitution de tout ou partie des protéines animales comprenant la poudre granulée selon la présente invention ou susceptible d'être obtenue selon la mise en oeuvre du procédé de préparation de poudre granulée selon l'invention tel que décrit ci-dessus.

Une des utilisations particulièrement avantageuse et intéressante de la présente invention comme substitut total ou partiel des protéines animales, et plus particulièrement des protéines laitières, concerne la réalisation d'un produit laitier choisi dans le groupe constitué par les fromages frais et affinés, les fromages tartinables, les laits fermentés, les smoothies au lait, les yaourts, les spécialités laitières, les glaces fabriquées à partir de lait.

Selon un mode préférentiel, la poudre selon l'invention est utilisée pour la fabrication de glaces avec une substitution totale ou partielle des protéines laitières, en les remplaçant par ladite poudre de la présente invention.

L'intérêt de cette application est exemplifié ci-après dans l'exemple 4.

Selon un autre mode plus préférentiel, la poudre selon l'invention est utilisée pour la fabrication de fromages avec substitution partielle ou totale des protéines laitières.

Dans la présente invention, le terme fromage désigne un aliment obtenu à partir de lait coagulé ou de produits laitiers, comme la crème, puis éventuellement d'un égouttage, suivi ou non d'une étape de fermentation et éventuellement d'un affinage (fromages affinés). La dénomination « fromage » est réservée, selon le décret n° 88-1206 du 30 décembre 1988, au produit fermenté ou non, affiné ou non, obtenu à partir de matières d'origine exclusivement laitière (lait entier, lait partiellement ou totalement écrémé, crème, matière grasse, babeurre), utilisées seules ou en mélange, et coagulées en totalité ou en partie avant égouttage ou après élimination partielle de leur eau.

Le lait est acidifié, généralement à l'aide d'une culture bactérienne. Une enzyme, la présure, ou un substitut comme par exemple de l'acide acétique, du vinaigre ou de la GDL (gluconodeltalactone), peut ensuite être adjointe afin de provoquer la coagulation et former le lait caillé et le petit-lait.

Dans la présente invention, le terme fromage désigne également tous les fromages fondus et tous les fromages fondus tartinables. Ces deux types de fromages sont obtenus par broyage, mélange, fonte et émulsification, sous l'effet de la chaleur et d'agents émulsifiants, d'une ou plusieurs variétés de fromage, avec ou sans adjonction de constituants laitiers et/ou d'autres denrées alimentaires (crème, vinaigre, épices, enzymes,..).

Une telle application est exemplifiée dans l'exemple 5 ci-après par l'essai concernant les fromages fondus tartinables.

Dans un autre mode préférentiel, la poudre selon l'invention est utilisée pour la fabrication de yaourts, en substitution totale ou partielle du lait, de la poudre de lait reconstituée ou des protéines de lait. Une telle application est exemplifiée dans l'exemple 6 ci-après.

Ainsi, la poudre granulée selon la présente invention ou susceptible d'être obtenue selon la mise en oeuvre du procédé de préparation de poudre granulée selon l'invention tel que décrit ci-dessus, peut être utilisée en substitution partielle ou totale des protéines laitières dans une formulation alimentaire appartenant au groupe défini par les fromages frais et affinés, les fromages fondus éventuellement tartinables, les laits fermentés, les smoothies au lait, les yaourts, les spécialités laitières, les glaces fabriquées à partir de lait.

Une autre des utilisations particulièrement avantageuse de la poudre selon la présente invention concerne la fabrication d'émulsions huile/eau très fines, et plus particulièrement la fabrication de coffee-whiteners.

Les blanchisseurs de café (ou de thé) ou « coffee whiteners » sont des émulsions huile/eau très fines destinées à être incorporées dans une boisson instantanée du type café ou thé comme alternative à certains produits laitiers, tels le lait ou encore la crème, ces derniers ayant une durée de conservation trop courte et étant trop chers. En effet, les blanchisseurs de café, qui existent aussi bien sous forme liquide que sous forme de poudre, ont une durée de conservation plus longue. Ils remplissent donc toutes les fonctions remplies par le lait ou la crème ajoutée au café et permettent de blanchir la boisson dans laquelle ils sont incorporés, donnant ainsi un aspect « café au lait ». Ils réduisent également l'amertume du café. Enfin, ils peuvent être utiles aux personnes ne digérant pas le lactose.

Un blanchisseur de café classique est composé :

- de sirop de glucose, qui sert de support ;
- de matière grasse, (comme par exemple l'huile de palme), qui est responsable de la viscosité et de l'effet blanchissant du produit, dû à la dispersion de la lumière sur la surface des globules gras formant l'émulsion ;
- d'émulsifiant (comme les mono et diglycérides) qui favorise la « mouillabilité » et la dispersibilité de la poudre dans un liquide chaud ;
- de caséinates de sodium qui sont des protéines contribuant notamment à l'effet blanchissant, et possédant des propriétés émulsifiantes et améliorant le goût du produit, notamment en réduisant le caractère âcre des acides tanniques par complexation avec ces derniers ;
- de sels stabilisants.

Les émulsions huile/eau sont généralement fabriquées sous la forme de poudres, par un procédé classique consistant à mélanger les divers constituants et former une émulsion, réaliser une étape d'homogénéisation suivie d'une étape optionnelle de pasteurisation et enfin réaliser le séchage par une étape d'atomisation.

Le résultat obtenu par l'étape d'homogénéisation est une réduction effective de la taille des particules à un niveau tel qu'il est possible de garantir une meilleure stabilité du produit. En effet, un des facteurs les plus importants dans la stabilité d'une émulsion est le diamètre des particules. L'opération d'homogénéisation a précisément pour but de réduire au maximum ce diamètre et en même temps de le rendre aussi uniforme que possible ; il en résulte alors une amélioration de la stabilité et un accroissement de la viscosité du milieu.

L'agent ou les agents émulsifiant(s) généralement utilisés pour la fabrication de ces émulsions huile dans eau en poudre sont les caséinates, en particulier les caséinates de sodium, éventuellement utilisés en présence de mono et/ou diglycérides.

Cependant, les caséinates de sodium sont très coûteux et de moins en moins disponibles sur le marché, et les producteurs de ces émulsions huile dans eau se voient dans l'obligation de trouver des substituts aux caséinates de sodium afin de pouvoir continuer à proposer aux consommateurs des produits bon marché.

La poudre granulée selon la présente invention est un excellent substitut aux caséinates de sodium dans l'application précitée, et est capable de conduire à des résultats équivalents en termes de stabilité, de finesse d'émulsion, ou encore de pouvoir blanchissant de ces émulsions huile dans eau.

De manière préférée, l'utilisation de la poudre granulée selon la présente invention en substitution partielle ou totale des caséinates de sodium dans la fabrication d'une émulsion huile/eau est encore plus satisfaisante et concluante lorsque le procédé suivant est appliqué :

- a) mélanger la matière grasse, le support et l'agent ou les agent(s) émulsifiant(s) sous forte agitation afin de réaliser une émulsion la plus fine et la plus stable possible. Généralement, on préfèrera dans un premier temps mélanger la matière grasse avec l'agent ou les agent(s) émulsifiant(s), puis ajouter les autres constituants sous forte agitation pour créer l'émulsion.
- b) pasteuriser optionnellement l'émulsion obtenue à l'étape a),
- c) parfaire l'émulsion par une étape d'homogénéisation de l'émulsion éventuellement pasteurisée obtenue à l'étape b),
- d) et transformer l'émulsion éventuellement pasteurisée et homogénéisée obtenue à l'étape c) en poudre, le plus souvent par atomisation, éventuellement combinée ou suivie d'une étape de granulation.

Ainsi, les performances obtenues dans la fabrication des émulsions huile/eau en poudre en utilisant la poudre granulée de la présente invention sont très sensiblement améliorées dans la mesure où dans le procédé de fabrication de ces émulsions huile/eau, l'étape optionnelle de pasteurisation est conduite avant l'étape d'homogénéisation, lorsque l'équipement technique industriel le permet.

Cela dit, des résultats tout à fait satisfaisants sont également obtenus lorsque le procédé dit classique est employé, à savoir lorsque l'étape d'homogénéisation est conduite avant l'étape optionnelle de pasteurisation.

En particulier, l'utilisation de ladite poudre granulée permet de répondre aux performances particulières et nécessaires recherchées pour les émulsions huile/eau utilisées comme blanchisseurs de café, à savoir une bonne dispersion dans une boisson chaude et éventuellement acide telle que le café, une excellente stabilité dans ladite boisson chaude et le même pouvoir blanchissant qu'un blanchisseur de café classique pour sensiblement la même quantité de produit incorporée à une boisson. La finesse d'émulsion est un paramètre important, et recherché pour ces émulsions huile dans eau en poudre, notamment pour l'effet blanchissant du café car plus les globules gras sont fins, plus la surface sur laquelle la lumière peut se disperser est grande, et donc plus l'effet blanchissant du produit est important. Un exemple d'utilisation de la poudre granulée selon la présente invention dans la préparation dune émulsion huile/eau en poudre pour une utilisation comme coffee whiteners est exemplifié ci-après.

La présente invention concerne en outre une émulsion huile/eau, et de préférence blanchisseurs de café ou de thé, comprenant la poudre granulée selon l'invention ou susceptible d'être obtenue selon la mise en oeuvre du procédé de préparation de poudre granulée selon l'invention tel que décrit ci-dessus.

Selon une autre variante, l'utilisation de ladite poudre granulée permet de répondre aux performances particulières et nécessaires recherchées pour les émulsions huile/eau en poudre ou liquide utilisées pour l'alimentation animale, et notamment pour l'alimentation des veaux.

Ainsi, notamment, de telles émulsions, préférentiellement en poudre, sont utilisées à titre de prémélanges gras pour l'alimentation animale, en particulier bovine, et notamment dans la préparation des aliments d'allaitement de veaux. En effet, il est également recherché des performances particulières pour les émulsions huile/eau en poudre utilisées comme prémélanges gras dans l'alimentation animale, en particulier pour l'allaitement des veaux, à savoir une bonne reconstitution de l'émulsion dans de l'eau tiède, de manière à obtenir facilement une émulsion liquide, une bonne stabilité de l'émulsion, c'est-à-dire sans déphasage de la phase huileuse et de la phase aqueuse de l'émulsion, une bonne appétence et un goût acceptable, voire agréable pour l'animal de manière à l'inciter à consommer ces prémélanges gras.

L'invention s'étend ainsi aux formulations alimentaires comprenant une poudre granulée selon l'invention ou susceptible d'être obtenue selon la mise en oeuvre du procédé de préparation de poudre granulée selon l'invention tel que décrit ci-dessus, ou comprenant un agent émulsifiant, foisonnant, stabilisant, épaississant et/ou gélifiant, pouvant être utilisé en substitution de tout ou partie des protéines animales tel que décrit ci-dessus, telles que :

- les boissons,
- les produits laitiers (dont par exemple les fromages frais et affinés, les fromages fondus éventuellement tartinables, les laits fermentés, les smoothies au lait, les yaourts, les spécialités laitières, les glaces fabriquées à partir de lait),
- les préparations destinées à la nutrition clinique et/ou à des individus souffrant de dénutrition,
- les préparations destinées à la nutrition infantile,

- les mélanges de poudres destinées à des produits de régime, ou pour sportifs,
- les soupes, sauces, et aides culinaires
- les produits à base de viande, plus particulièrement dans les secteurs des pâtes fines et des saumures, notamment dans la fabrication de jambons,
- les produits à base de poissons, comme les produits à base de surimi,
- tous les types de confiseries,
- les produits céréaliers comme le pain, les pâtes, biscuits, pâtisseries, céréales et barres,
- les produits végétariens et plats cuisinés.

La poudre granulée selon la présente invention ou susceptible d'être obtenue selon la mise en oeuvre du procédé de préparation de poudre granulée selon l'invention tel que décrit ci-dessus trouve également des applications en alimentation animale.

L'invention sera encore mieux comprise à la lecture des exemples qui suivent, lesquels se veulent illustratifs en faisant seulement état de certains modes de réalisation et de certaines propriétés avantageuses selon l'invention, et non limitatifs.

**EXEMPLE 1 :** Préparation d'une poudre granulée selon l'invention

Une poudre granulée contenant 45% de protéines de pois et 55% de maltodextrines de DE 19 a été préparée de la manière suivante.

Les protéines de pois utilisées sont commercialisées par la demanderesse sous la dénomination Nutralys® S 85 M. Leur taux de protéines totales est de 85%.

Les maltodextrines utilisées appartiennent à la gamme GLUCIDEX®, également commercialisée par la Demanderesse, et sont les maltodextrines GLUCIDEX® de DE 19.

- Tout d'abord, une suspension a été préparée à un ratio protéines/maltodextrines de 45/55 dans une cuve agitée et à une température de 50°C.
- Le mélange a une MS de 35%.
- Le mélange obtenu a été homogénéisé sur un homogénéisateur haute pression à doubles étages (150 bars sur le 1$^{er}$ étage et 50 bars sur le second) avant d'être atomisé, et ce afin d'avoir un mélange parfaitement homogène.
- Le mélange a été atomisé dans une tour d'atomisation de type MSD équipée d'une buse d'atomisation haute pression avec recyclage des fines particules en tête de tour.

Les conditions d'atomisation sont les suivantes :

- La buse d'injection a été choisie de manière à obtenir une pression de 220 bar pour un débit de 120 1/h.
- L'air utilisé était à 6g/kg d'humidité.
- La température des airs d'entrée ont été réglées de la manière suivante :

  - pour l'air d'entrée en amont de la tête de tour : température de 180 °C,
  - pour le lit fluidisé statique : température de 50/55°C,
  - pour le lit fluidisé vibré : température de l'ordre de 20°C.

- La température de sortie était de 58°C.
- la vitesse de l'air en amont a été réglée à 14,7 m/s, et celle de l'air du lit fluidisé statique était de 11 m/s.

La poudre granulée obtenue selon l'exemple 1 présentait les caractéristiques suivantes.

- Humidité : 7%

- Matière sèche : 93%

- Diamètre moyen volumique D 4,3 : 200 $\mu$m

**EXEMPLE 2:** Mesure du pouvoir gélifiant

Le pouvoir gélifiant de la poudre granulée obtenue selon l'exemple 1 a été comparé au pouvoir gélifiant du simple mélange de poudre, en utilisant les deux même constituants, ainsi que le même ratio, que ceux utilisés pour la préparation de la poudre granulée.

1. Préparation des solutions

Une solution à 8% en concentration a été préparée en disposant 8g d'échantillon (poudre granulée ou simple mélange de poudres) dans 100 g d'eau distillée à 20°C +/- 1°C. 0,3g de gomme xanthane ont été ajoutés aux solutions précédentes afin d'éviter la décantation des particules sous l'effet de la gravité. Le mélange a été placé sous agitation lente pendant 30 min à une vitesse de 250 rpm afin de permettre une hydratation optimale des protéines contenues dans les échantillons.

2. Matériel de mesure

Une caractérisation de la gélatinisation des échantillons au cours d'un cycle thermique a été réalisée, en mode dynamique oscillatoire, au moyen du rhéomètre PHYSICA® MCR301 (Anton Paar) équipé d'une géométrie de type plateaux parallèles striés afin d'éviter les phénomènes de glissement.

3. Protocole de mesure

1 ml de la suspension hydratée, préparée dans le paragraphe (1), placé entre les plateaux parallèle de diamètre 50 mm, a été soumis à une sollicitation de type sinusoïdale, à la fréquence de 1 Hertz et une amplitude de déformation de 0.1 à 0.5 %, tout en appliquant le cycle thermique suivant :

1. Chauffage de 20 à 90°C en 2000s -0.5% de déformation,
2. Maintien à 90°C pendant 3600s - 0.2 % de déformation,
3. Refroidissement de 90 à 4°C en 2000s - 0.1% de déformation,
4. Maintien à 4°C pendant 12000s - 0.1 % de déformation.

4. Interprétation

Le suivi des niveaux de modules de conservation G' et de dissipation G" a permis de caractériser la cinétique de la gélification de la protéine sous l'effet de la chaleur ainsi que le niveau relatif de la force du gel obtenu.
Les courbes obtenues ont permis de mesurer la vitesse de gélification, ainsi que la force du gel obtenu mais également le comportement du gel à froid.
Les courbes obtenues avec la poudre granulée en comparaison des courbes obtenues avec le simple mélange présentaient une vitesse de gélification plus rapide, un niveau maximum plus élevé ce qui signifie que les gels étaient plus solides, ainsi qu'une meilleure texture et tenue du gel à froid.
Ceci signifie que le pouvoir gélifiant de la poudre granulée était bien meilleur que le pouvoir gélifiant du simple mélange physique.

**EXEMPLE 3:** Préparation d'une émulsion huile/eau (coffee whiteners)

Dans cet exemple, la poudre granulée obtenue selon l'exemple 1 a été utilisée pour la préparation d'une émulsion huile/eau en poudre, et a été comparée à une poudre témoin à base de caséinate de sodium.

• Ingrédients de l'émulsion comprenant la poudre granulée selon l'invention (en pourcentage d'ingrédients mis en oeuvre)

  ◦ 61,65% de support (Roquette sirop de glucose 3072)
  ◦ 30% de matière grasse (huile de palme)
  ◦ 5,55% de poudre granulée obtenue selon l'exemple 1
  ◦ 2% de sels stabilisants (di-potassium hydrogenophosphate)
  ◦ 0.8% de mono et diglycérides.

• Ingrédients de l'émulsion témoin comprenant des caséinate de sodium (en pourcentage d'ingrédients mis en oeuvre)

- 64,7% de support (Roquette sirop de glucose 3072)
- 30% de matière grasse (huile de palme)
- 2.5% de caséinate de sodium
- 2% de sels stabilisants (di-potassium hydrogenophosphate)
- 0.8% de mono et diglycérides.

• Procédé de fabrication des émulsions huile/eau en poudre

a) L'eau et le sirop de glucose ont été mélangés et portés à 65°C dans un bécher placé dans un bain-marie.

Parallèlement, l'huile de palme a été fondue dans un autre bécher à une température de 65°C. Les mono et diglycérides ont été dispersés dans l'huile lors de sa fonte.

Lorsque le mélange eau/sirop de glucose a atteint la température voulue, les produits en poudres (poudre granulée selon l'invention ou caséinates de sodium, sels stabilisants) y ont été ajoutés et le tout a été mélangé grâce à un agitateur de type KENWOOD®, à une vitesse de 10 000 tours/min.

L'huile de palme fondue contenant les mono et diglycérides a été ajoutée progressivement au mélange eau/sirop de glucose/poudres, sous agitation grâce à un agitateur de type POLYTRON®, à une vitesse de 4000 tours/min.

La composition résultante présentait une teneur en eau de 50%.

b) Le mélange a été pasteurisé à 80°C pendant une dizaine de secondes, de manière à éliminer les bactéries susceptibles de se développer dans le produit mais également, afin d'augmenter la stabilité de l'émulsion qui sera obtenue à la fin du procédé.

c) Ce mélange pasteurisé a été homogénéisé grâce à un homogénéisateur NIRO® Soavi (groupe GEA).

Le premier étage a été réglé à une pression de 170 bars et le deuxième à une pression de 30 bars.

L'observation de la stabilité de ces émulsions a été réalisée avant atomisation. Pour cela l'émulsion a été laissée pendant une heure sans agitation à température ambiante.

Si un déphasage a été observé, l'émulsion n'était pas stable.

La granulométrie (taille des globules gras) de l'émulsion avant atomisation a été mesurée grâce à un granulomètre laser BECKMAN COULTER® couplé à un ordinateur. Cet appareil permet de mesurer la répartition de la taille des globules gras.

d) L'émulsion ainsi obtenue a été atomisée. L'atomisation a été réalisée dans un atomiseur à une température d'air entrant dans l'atomiseur de 200°C et une température du produit à la sortie de 95°C.

Les émulsions huile/eau en poudre ainsi obtenues ont été caractérisées par :

- La mesure de la taille des globules gras de ces émulsions par granulométrie laser comme précisé ci-avant.
- Le test de reconstitution de ces émulsions dans de l'eau à 80°C et du café à 80°C (café soluble Nescafé® et café filtre Carte Noire®) et observation de la stabilité de ces émulsions reconstituées (précipitation ou non des protéines). Pour cela l'émulsion reconstituée a été laissée pendant une heure sans agitation à température ambiante.
- La mesure du pouvoir blanchissant de ces émulsions par reconstitution de ces émulsions dans du café à 80°C (café soluble Nescafé® et café filtre Carte Noire®) et mesure par un colorimètre.
- L'évaluation du goût de ces émulsions par un panel d'évaluateurs en analyse sensorielle.

• Résultats

- mesure de la taille des globules gras

La taille des globules gras obtenue dans les deux émulsions (l'émulsion réalisée avec la poudre granulée et l'émulsion témoin) était sensiblement la même. Les émulsions obtenues étaient relativement fines avec une taille moyenne des globules gras d'environ 2μm.

- test de reconstitution et observation de la stabilité

On a observé que l'émulsion huile/eau en poudre comprenant la poudre granulée de la présente invention ne déphasait pas dans l'eau à 80°C et dans le café à 80°C (café soluble Nescafé® et café filtre Carte Noire®), et possédait le même comportement que l'émulsion huile/eau témoin comprenant les caséinates de sodium.

- Mesure du pouvoir blanchissant

L'émulsion huile/eau en poudre comprenant la poudre granulée de la présente invention possédait un pouvoir blanchissant du même ordre que l'émulsion témoin huile/eau en poudre à base de caséinate de sodium.

- Evaluation du goût

L'émulsion huile/eau en poudre à base de caséinate de sodium possédait un goût jugé très acceptable par le panel d'évaluateurs en analyse sensorielle.
L'émulsion huile/eau en poudre comprenant la poudre granulée de la présente invention possédait un goût jugé acceptable par le panel d'évaluateurs en analyse sensorielle.

• Conclusion

L'émulsion huile/eau en poudre comprenant la poudre granulée de la présente invention possède des caractéristiques similaires à l'émulsion huile/eau en poudre à base de caséinate de sodium. Ces caractéristiques des émulsions sont nécessaires pour une utilisation comme blanchisseur de café.
Ainsi, la poudre granulée de la présente invention, comprenant au moins une protéine végétale et au moins un hydrolysat d'amidon est un bon substitut au caséinate de sodium dans les émulsions huile/eau en poudre utilisées comme blanchisseur de café.

Variante de l'exemple 3

L'exemple 3 a été reproduit de façon identique à celle décrite dans l'exemple 3, en remplaçant le support (Roquette sirop de glucose 3072) par des maltodextrines de type GLUCIDEX® 19, commercialisées par la société Roquette, et l'huile de palme par de l'huile de coco. Les résultats obtenus ont été aussi intéressants que ceux précédemment obtenus.

**EXEMPLE 4:** Préparation de glaces aromatisées avec substitution totale des protéines laitières

Dans cet exemple, la poudre granulée a été obtenue selon le protocole mis en oeuvre dans l'exemple 1 en utilisant cette-fois un ratio pondéral composition de protéines de pois/maltodextrines de 70/30.
La poudre granulée contient donc 70% d'une composition de protéines de pois (à un taux de protéines totales de 85%) et 30% de maltodextrines de DE 19.
Les crèmes glacées ont été préparées selon les recettes figurant dans le tableau ci-dessous et les produits finaux ont été goûtés, notés et comparés par un jury d'analyse sensoriel.
Deux recettes de glace ont été testées, l'une au caramel et l'autre au chocolat.
4 échantillons ont donc été obtenus :

- TEMOIN 1: Glace arôme caramel préparée à partir de lait entier et aromatisée avec un arôme caramel (Symrise, réf. 186745),
- ESSAI 1 : Glace arôme caramel aromatisée avec un arôme caramel (Symrise, réf. 186745), et ne contenant plus de lait mais contenant la poudre granulée selon l'invention,
- TEMOIN 2 : Glace arôme chocolat préparée avec du lait entier, de la poudre de chocolat et aromatisée avec un arôme chocolat (Symrise, réf. 225962),
- ESSAI 2 : Glace arôme chocolat aromatisée avec un arôme chocolat (Symrise, réf. 225962), et ne contenant plus de lait mais contenant la poudre granulée selon l'invention,

1. Recettes

| Ingrédients (%) | Glace arôme caramel TEMOIN 1 | Glace arôme caramel ESSAI 1 | Glace arôme chocolat TEMOIN 2 | Glace arôme chocolat TEMOIN 2 |
|---|---|---|---|---|
| Eau | 0 | 59,75 | 0 | 59, 75 |
| Lait entier | 69, 75 | 0 | 69,75 | 0 |
| Poudre de chocolat | 0 | 0 | 4 | 4 |
| Arôme caramel 186745 | 1 | 1 | 0 | 0 |
| Arôme chocolat 225962 | 0 | 0 | 1 | 1 |
| Saccharose | 12 | 12 | 10 | 10 |
| Sirop de glucose DE 40, 80% MS | 8 | 8 | 6 | 6 |
| Huile de coco (végétaline) | 9 | 9 | 9 | 9 |
| Poudre granulée de l'invention | 0 | 10 | 0 | 10 |
| Emulsifiant 709 VEG | 0,25 | 0,25 | 0,25 | 0,25 |
| TOTAL | 100 | 100 | 100 | 100 |

2. Mode opératoire

- Mélanger les poudres à sec, peser l'émulsifiant en dernier en le mélangeant au sucre.

- Disperser le mélange de poudres dans le lait entier à 45°C (témoins 1 et 2) ou dans l'eau (essais 1 et 2) à 45°C pendant 20 minutes.

- Incorporer ensuite les matières grasses et le sirop de glucose.

- Laisser en agitation pendant 15 minutes.

- Pasteuriser à 80°C durant 3 minutes, refroidir puis passer le mix (température comprise entre 65 et 70°C) sur un homogénéisateur à une pression de 250 bars.

- Ajouter l'arôme et laisser maturer avec une agitation lente à une température de 4°C pendant 6 heures minimum.

- Foisonner à 100% et congeler dans le freezer.

- Surgeler à -30°C pendant 2 heures.

- Stocker à -20°C.

3. Tests d'analyse sensorielle

Les échantillons de glace au caramel et au chocolat ont été goutés en aveugle par un jury d'experts en analyse sensoriel de 20 personnes.

Le premier test consistait en un test triangulaire où, sur les trois échantillons proposés, deux étaient identiques. 75% des personnes ayant participés au test n'ont pas pu reconnaître quels étaient les deux échantillons identiques, et ce pour les deux arômes testés.

Aucun des échantillons testés n'a reçu une préférence significative au niveau du jury.

Le second test, toujours réalisé en aveugle, consistait à goûter les différents échantillons et à les décrire. Les qualificatifs employés étaient identiques pour les glaces contenant des protéines de lait et pour celles n'en contenant pas (onctuosité, rondeur en bouche, crémeux).

Ces deux séries de tests d'analyse sensorielle démontrent parfaitement que le jury entrainé n'a pas su faire de différence entre une glace au lait et une glace ne contenant plus de protéines laitières mais une poudre granulée selon la présente invention, ou susceptible d'être obtenue selon la mise en oeuvre du procédé de préparation de poudre granulée selon l'invention tel que décrit ci-dessus.

Cette invention va permettre notamment aux personnes allergiques aux protéines laitières de pouvoir déguster des crèmes glacées aussi bonnes et onctueuses que leurs équivalents contenant du lait.

**EXEMPLE 5:** Préparation de fromage fondu tartinable avec substitution de 10% de protéines laitières

Dans cet exemple, la poudre granulée identique à celle de l'exemple 4 précédent a été utilisée.

Cette poudre granulée contenait donc 70% d'une composition de protéines de pois (à un taux de protéines totales de 85%) et 30% de maltodextrines de DE 19.

Le fromage fondu (ESSAI) a été préparé selon la recette figurant dans le tableau ci-dessous, et contient la poudre granulée de ladite invention. Il a ensuite été comparé à un fromage témoin (TEMOIN) préparé en parallèle et dans les mêmes conditions et ne contenant pas la poudre granulée selon la présente invention.

1. Recettes

|  | TEMOIN | ESSAI |
|---|---|---|
| **Cheddar (70% M.S.)** | 32,73 | 31,70 |
| **Caséine présure** | 7, 14 | 5,70 |
| **Beurre** | 9, 06 | 9,06 |
| **Sel de fonte (Joha S9)** | 1,20 | 1,20 |
| **Sel de fonte (Joha S4)** | 1,20 | 1,20 |
| **Sel de fonte (Joha Tneu)** | 0, 13 | 0, 13 |
| **Poudre granulée selon l'invention** | 0 | 3,20 |
| **Eau** | 48,54 | 48,50 |
| **TOTAL** | 100 | 100 |
| **Taux de substitution des protéines laitières** | - | 10,4 |

2. Mode opératoire

- Préchauffage de la double enveloppe du cuiseur par injection de vapeur (Stéfan) jusqu'à 100°C.
- Ajout des ingrédients et agitation à 300 rpm pendant 30 secondes.
- Agitation à 3000 rpm en chauffant jusqu'à 95°C. Maintien 3 minutes à 95°C.
- Conditionnement en barquettes et maintien à température ambiante pendant 24 heures.
- Refroidissement et conservation à 4°C.

3. Valeurs nutritionnelles des deux fromages

| Valeur nutritionnelle sur 100g de produit (à la mise en oeuvre) | TEMOIN | ESSAI |
|---|---|---|
| **Protéines totales** | 14,4 | 14,3 |
| **Dont protéines laitières** | 14,4 | 12,9 |

(suite)

| Valeur nutritionnelle sur 100g de produit (à la mise en oeuvre) | TEMOIN | ESSAI |
|---|---|---|
| Dont protéines végétales | 0 | 1,4 |
| Glucides | 0, 47 | 1, 14 |
| Lipides | 18,5 | 18,2 |

4. Autres paramètres

| | TEMOIN | ESSAI |
|---|---|---|
| pH | 6,1 | 6,2 |
| Matière Sèche théorique | 40, 90 | 37, 60 |
| Humidité sur fromage dégraissé | 74,20 | 76, 40 |

L'exemple ci-dessus démontre parfaitement qu'il est tout à fait possible de remplacer une partie des protéines laitières par la composition de la présente invention sans pour autant modifier les valeurs nutritionnelles de façon significative. Au niveau du goût, les deux fromages ont été goûtés par un jury entrainé de 20 personnes et ont été jugés comme similaires et très satisfaisants.

**EXEMPLE 6:** Préparation de yaourts contenant la poudre granulée selon la présente invention

Dans une première série de tests, des essais ont été réalisés en substituant le lait par la poudre granulée ou par le mélange simple des deux constituants. Deux pourcentages de substitution ont été testés : 10% et 50%.
Dans une seconde série de tests, la substitution du lait dans des yaourts par une poudre granulée selon la présente invention a été réalisée à des pourcentages de substitution variables : 10, 20, 30, 40 et 50%.
Enfin, dans une troisième et dernière série d'essais, la substitution du lait à un taux de 50% a été testée pour des poudres granulées selon la présente invention qui présentent des ratios pondéraux pois/maltodextrines variables. Trois ratios pondéraux pois/maltodextrines ont été testés : 45/55, 30/70 et 60/40.
La texture, la couleur et le goût ont été les paramètres mesurés et comparés pour les trois séries d'essais.
Le mode opératoire de préparation des différents yaourts a été le même dans les trois séries d'essais, et consiste en :

- Mise en solution des poudres dans le lait préchauffé à 50°C.
- Passage sur l'homogénéisateur haute pression de type NIRO® Soavi (groupe GEA) pendant quelques secondes.
- Etape de pasteurisation du mélange à 90°C pendant 20 minutes.
- Conditionner dans des flacons de 500 ml et laisser refroidir au bain-marie jusque 42°C.
- Incorporer le ferment préalablement préparé : dans 200 ml incorporer un sachet de ferment et le laisser sous agitation pendant 30 minutes ajouter 1 ml de ferment pour 500 ml de solution.
- Laisser fermenter le tout jusqu'à obtention d'un pH de 4,5.
- Lissage de la solution et conditionnement en sortie.
- Réserver à 4°C.
- Mesurer le pH, la viscosité et la blancheur de chaque échantillon.

A. Première série de tests

Dans cette série, la poudre granulée a été obtenue selon l'exemple 1 en utilisant un ratio pondéral protéines de pois/maltodextrines de 45/55.
Plus précisément la composition de protéines de pois contenait 85% de protéines totales de pois et les maltodextrines sont de DE 19. Parallèlement le mélange simple des deux constituants a été réalisé en respectant les mêmes proportions pour les deux constituants que celles utilisées pour l'obtention de la poudre granulée.

• Recettes utilisées

| Taux de substitution | | Poudre granulée | | Mélange « simple » | |
|---|---|---|---|---|---|
| | | 10% | 50% | 10% | 50% |
| | TEMOIN | E10 | E50 | T10 | T50 |
| lait écrémé du commerce | 86,8 | 82, 17 | 45, 65 | 82, 17 | 45, 65 |
| Lait écrémé en poudre reconstitué à 10% | 4,5 | / | / | / | / |
| Poudre granulée selon l'invention en solution à 10% | / | 4, 63 | 41,15 | / | / |
| Poudre granulée selon l'invention en poudre | / | 4,5 | 4,5 | / | / |
| Mélange simple en solution | / | / | / | 4, 63 | 41,15 |
| Mélange simple en poudre | / | / | / | 4,5 | 4,5 |
| CLEARAM® CH2020 | 0,4 | 0,4 | 0,4 | 0,4 | 0,4 |
| Gélatine 200 Blooms-type A (40% MS) | 0,3 | 0,3 | 0,3 | 0,3 | 0,3 |
| Sucrose | 8 | 8 | 8 | 8 | 8 |
| Ferment YC380 | qsp | qsp | qsp | qsp | qsp |
| Arôme masquant | 0, 07 | 0,07 | 0, 07 | 0, 07 | 0,07 |
| Total | 100 | 100 | 100 | 100 | 100 |

Le ferment pour yaourt utilisé est commercialisé par la société CHR Hansen A/S (Danemark) sous la référence CH-YC 380.

La gélatine utilisée provient de la société Rousselot SAS, Courbevoie (France).

Le CLEARAM® CH2020 est commercialisé par la société Demanderesse et se définit comme un amidon modifié à cuire.

• Résultats

| | Témoin | E10 | E50 | T10 | T50 |
|---|---|---|---|---|---|
| pH | 4,6 | 4,3 | 4, 4 | 4,4 | 4,5 |
| Texture | Légèrement gélifié, lisse. | Gélifié lisse. | Gélifié lisse. | Liquide granuleux. | Liquide granuleux. |
| Couleur | Blanc | Blanc cassé | Blanc cassé | Blanc cassé | Blanc cassé |

Cette première série de tests démontrent d'une part que le simple mélange physique des deux poudres de protéines de pois et de maltodextrine ne permet pas d'obtenir une texture ferme dans une recette de yaourt. Autrement dit, il n'y a pas de gélification et le yaourt reste liquide avec une texture granuleuse. Par contre la poudre granulée selon la présente invention permet d'obtenir un yaourt gélifié à texture lisse et ce aux deux pourcentages de substitution du lait testé : 10 et 50%.

Les quatre yaourts contenant des protéines de pois ont une couleur légèrement plus blanc cassé que la couleur blanche du yaourt témoin, mais cela reste très minime.

Cet essai démontre parfaitement d'une part qu'il est possible de fabriquer des yaourts en substituant jusqu'à 50% de lait par la poudre granulée de la présente invention, ou susceptible d'être obtenue selon la mise en oeuvre du procédé de préparation de poudre granulée selon l'invention tel que décrit ci-dessus, et d'autre part que ladite poudre possède des propriétés fonctionnelles gélifiantes qui lui ont été conférées notamment par son procédé de préparation, propriétés qui ne sont pas retrouvées sur le mélange simple des constituants.

B. Seconde série de tests

Dans cette série, la poudre granulée était identique à celle utilisée dans la première série de tests (ratio pondéral protéines de pois/maltodextrines (de DE 19) de 45/55). A chaque fois, la composition de protéines de pois utilisées pour l'obtention de la poudre granulée contenait 85% de protéines de pois.

• Recettes utilisées

TABLEAU 1

| | TEMOIN | POUDRE GRANULEE SELON L'INVENTION | | | | |
|---|---|---|---|---|---|---|
| % de substitution | | 10% | 20% | 30% | 40% | 50% |
| Solution lait écrémé à 10% | 86,8 | 82, 17 | 73,04 | 63,91 | 54,78 | 45, 65 |
| Lait écrémé en poudre | 4,5 | / | / | / | / | / |
| Poudre granulée selon l'invention en solution à 10% | / | 4, 63 | 13,76 | 22,89 | 32,02 | 41, 15 |
| Poudre granulée selon l'invention en poudre | / | 4,5 | 4,5 | 4,5 | 4,5 | 4,5 |
| CLEARAM® CH2020 | 0,4 | 0,4 | 0,4 | 0,4 | 0,4 | 0,4 |
| Gélatine 200 Blooms-type A (40% MS) | 0,3 | 0,3 | 0,3 | 0,3 | 0,3 | 0,3 |
| Sucrose | 8 | 8 | 8 | 8 | 8 | 8 |
| Ferment YC380 | qsp | qsp | qsp | qsp | qsp | qsp |
| Total | 100 | 100 | 100 | 100 | 100 | 100 |

• Résultats

| | Témoin | Poudre Granulée | | | | |
|---|---|---|---|---|---|---|
| | | 10% | 20% | 30% | 40% | 50% |
| pH | 4,5 | 4,4 | 4, 4 | 4,3 | 4,3 | 4,3 |
| Texture | Gélifié lisse | Gélifié lisse. | Gélifié lisse. | Gélifié lisse | Gélifié lisse | Gélifié lisse |
| Couleur | Blanc | Blanc cassé | Blanc cassé | Blanc cassé | Blanc cassé | Blanc cassé |

Cette seconde série de tests démontrent qu'il est tout à fait possible de substituer les protéines de lait dans un yaourt par la poudre granulée de la présente invention, ou susceptible d'être obtenue selon la mise en oeuvre du procédé de préparation de poudre granulée selon l'invention tel que décrit ci-dessus. Les taux de substitution peuvent aller jusqu'à 50%. Les cinq taux de substitution donnent des yaourts tout à fait satisfaisants et comparables au yaourt témoin au niveau de la texture, qui reste gélifiée et lisse. La couleur des cinq yaourts essais est très légèrement blanc cassé mais cela reste très minime.

C. Troisième série de tests

Dans cette série, la substitution du lait dans un yaourt à un taux de 50% est testée pour des poudres granulées selon la présente invention présentant des ratios pondéraux pois/maltodextrines variables : 30/70, 45/55 et 60/40. Pour chaque poudre granulée testée, la composition de protéines de pois utilisées contient 85% de protéines de pois, et la maltodextrine est toujours une maltodextrine possédant un DE de 19.

Recettes utilisées

| | TEMOIN | POUDRE GRANULEE SELON L'INVENTION | | |
|---|---|---|---|---|
| Ratio pondéral protéines pois / maltodextrines | | 30/70 | 45/55 | 60/40 |
| Solution lait écrémé à 10% | 86,8 | 45, 65 | 45, 65 | 45, 65 |
| Lait écrémé en poudre | 4,5 | / | / | / |
| Poudre granulée selon l'invention en solution à 10% | / | 41, 15 | 41, 15 | 41,15 |

(suite)

| | TEMOIN | POUDRE GRANULEE SELON L'INVENTION | | |
|---|---|---|---|---|
| **Poudre granulée selon l'invention en poudre** | / | 4,5 | 4,5 | 4,5 |
| **CLEARAM® CH2020** | 0,4 | 0,4 | 0,4 | 0,4 |
| **Gélatine 200 Blooms-type A (40% MS)** | 0,3 | 0,3 | 0,3 | 0,3 |
| **Sucrose** | 8 | 8 | 8 | 8 |
| **Ferment YC380** | qsp | qsp | qsp | qsp |
| **Arôme + colorant** | qsp | qsp | qsp | qsp |
| **Total** | 100 | 100 | 100 | 100 |

• Résultats

| | TEMOIN | POUDRE GRANULEE SELON L'INVENTION | | |
|---|---|---|---|---|
| **Ratio pondéral protéines pois/ maltodextrines** | | 30/70 | 45/55 | 60/40 |
| | | | | |
| **pH** | 4,6 | 4,3 | 4,5 | 4,5 |
| **Viscosité (m/Pas)** | 16 000 | 11 200 | 56 000 | 58 000 |
| **Texture** | Gélifié, lisse | Légèrement gélifié, lisse | Gélifié, lisse | Gélifié, lisse |
| **Couleur** | Blanc | Légèrement teinté | Légèrement teinté | Plutôt beige |

Cette troisième série de tests démontrent que la poudre granulée présentant un ratio pondéral composition de protéines de pois/maltodextrines de 45/55 (avec une teneur de 85% de protéines de pois dans la composition et une maltodextrine de DE 19) est la poudre qui permet d'obtenir un yaourt s'approchant le plus du yaourt témoin. Lorsqu'on augmente la teneur de la composition de protéines de pois dans le ratio (60/40), le yaourt obtenu est très crémeux mais la coloration est légèrement beige, ce qui peut-être un handicap. A un ratio inférieur (30/70), la texture du yaourt est moins gélifiée que le yaourt témoin.

**EXEMPLE 7:** Préparation de yaourts à boire contenant la poudre granulée selon la présente invention

Dans cet exemple, la poudre granulée était identique à celle des séries 1 et 2 de l'exemple 6 précédent. Le ratio pondéral protéines de pois/maltodextrines (de DE 19) était donc de 45/55. A chaque fois, la composition de protéines de pois utilisées pour l'obtention de la poudre granulée contenait 85% de protéines de pois.

Des essais ont été réalisés en substituant le lait par la poudre granulée ou par le mélange simple des deux constituants. Deux pourcentages de substitution ont été testés : 10% et 50%.

Les yaourts à boire (ESSAI 10 et ESSAI 50) ont été préparés selon la recette figurant dans le tableau ci-dessous, et contiennent la poudre granulée de ladite invention aux deux taux de substitution différents. Ils ont ensuite été comparés au yaourt ne contenant que du lait ainsi qu'aux yaourts à boire témoin (TEMOIN 10 et TEMOIN 50) préparés en parallèle, dans les mêmes conditions et ne contenant pas la poudre granulée selon la présente invention mais le simple mélange physique des deux constituants.

Les différents yaourts à boire ont été dégustés en aveugle par un jury entrainé et expert en analyse sensoriel de 20 personnes. Les paramètres suivants ont été testés et notés sur une échelle de 1 à 5, 1 étant la note la plus mauvaise et 5 la meilleure : couleur, odeur, goût, onctuosité en bouche, consistance, note générale.

1. Recettes utilisées

| Taux de substitution | | | Poudre granulée | | Mélange « simple » | |
|---|---|---|---|---|---|---|
| | | | 10% | 50% | 10% | 50% |
| | | TEMOIN | E10 | E50 | T10 | T50 |
| lait écrémé du commerce | | 86 | 77,5 | 43 | 77,5 | 43 |
| Poudre granulée selon l'invention en poudre | | / | 8,5 | 43 | 8,5 | 43 |
| SweetPearl™ P200 | | 10 | 10 | 10 | 10 | 10 |
| NUTRIOSE® FB06 | | 4 | 4 | 4 | 4 | 4 |
| Ferment probiotique BMY-1 | | qsp | qsp | qsp | qsp | qsp |
| Total | | 100 | 100 | 100 | 100 | 100 |

Le SweetPearl™ P200 est le nom commercial du maltitol de la société Demanderesse : il s'agit d'un glucide sous forme de poudre cristalline, issu de l'amidon de blé et de maïs.

Le NUTRIOSE® FB06 est une fibre soluble commercialisée également par la société Demanderesse.

Le ferment utilisé est commercialisé par la société CHR hansen A/S (Danemark).

2. Mode opératoire

• Le NUTRIOSE® FB06 a été dissous dans le lait.

• Le mélange a ensuite été pasteurisé à 90°C pendant 10 minutes.

• Ce mélange pasteurisé a ensuite été homogénéisé grâce à un homogénéisateur NIRO® Soavi (groupe GEA), à une pression de 180 bars.

• L'émulsion ainsi obtenue a ensuite été refroidie à 43°C et maintenue à cette température.

• Les ferments prébiotiques ont été rajoutés au mélange refroidi, et la fermentation a été contrôlée en mesurant continuellement le pH à l'aide d'un pH-mètre.

• La fermentation a été stoppée lorsque le pH du mélange a atteint la valeur de 4,5.

• Le SweetPearl a ensuite été rajouté et le mélange a été homogénéisé avec un homogénéisateur de type ALM2, commercialisé par la société PIERRE GUERIN TECHNOLOGIES (France).

• L'ensemble a été pasteurisé à 90°C pendant 15 secondes, afin d'éliminer les risques de contamination microbiologique.

• Le tout a été refroidi à 5°C avant dégustation.

3. Résultats

| | | Poudre granulée | | Mélange « simple » | |
|---|---|---|---|---|---|
| | | 10% | 50% | 10% | 50% |
| | TEMOIN | E10 | E50 | T10 | T50 |
| Couleur | 5 | 4 | 4 | 3 | 4 |
| Goût | 5 | 5 | 4 | 3 | 2 |

(suite)

|  |  | Poudre granulée | | Mélange « simple » | |
|---|---|---|---|---|---|
|  |  | 10% | 50% | 10% | 50% |
|  | TEMOIN | E10 | E50 | T10 | T50 |
| **Onctuosité en bouche** | 5 | 5 | 4 | 2 | 2 |
| **Consistance** | 5 | 5 | 5 | 2 | 1 |
| **Note générale** | 5 | 5 | 4 | 2 | 2 |

Cet essai démontre parfaitement qu'il est tout à fait possible de substituer une partie des protéines laitières dans un yaourt à boire par la poudre granulée de la présente invention, ou susceptible d'être obtenue selon la mise en oeuvre du procédé de préparation de poudre granulée selon l'invention tel que décrit ci-dessus.

Les yaourts à boire contenant ladite poudre granulée ont été jugés très satisfaisants et identiques au yaourt à boire témoin ne contenant que du lait, avec une très légère préférence mais non significative pour le yaourt à boire E10 (substitution des protéines de lait à un taux de 10%). Les deux yaourts à boire contenant les simples mélanges physiques des deux constituants ont été jugés négativement et leur évaluation démontre parfaitement qu'ils ne se rapprochent absolument pas du yaourt témoin, aussi bien au niveau du goût, de leur onctuosité en bouche et de leur consistance (jugés beaucoup trop liquides). Ainsi, ladite poudre possède des propriétés fonctionnelles gélifiantes qui lui ont été conférées notamment par son procédé de préparation, propriétés qui ne sont pas retrouvées sur le mélange simple des constituants.

Cette troisième série de tests démontrent que la poudre granulée présentant un ratio pondéral composition de protéines de pois/maltodextrines de 45/55 (avec une teneur de 85% de protéines de pois dans la composition et une maltodextrine de DE 19) est la poudre qui permet d'obtenir un yaourt s'approchant le plus du yaourt témoin. Lorsqu'on augmente la teneur de la composition de protéines de pois dans le ratio (60/40), le yaourt obtenu est très crémeux mais la coloration est légèrement beige, ce qui peut-être un handicap. A un ratio inférieur (30/70), la texture du yaourt est moins gélifiée que le yaourt témoin.

**EXEMPLE 7:** Préparation de yaourts à boire contenant la poudre granulée selon la présente invention

Dans cet exemple, la poudre granulée était identique à celle des séries 1 et 2 de l'exemple 6 précédent. Le ratio pondéral protéines de pois/maltodextrines (de DE 19) était donc de 45/55. A chaque fois, la composition de protéines de pois utilisées pour l'obtention de la poudre granulée contenait 85% de protéines de pois.

Des essais ont été réalisés en substituant le lait par la poudre granulée ou par le mélange simple des deux constituants. Deux pourcentages de substitution ont été testés : 10% et 50%.

Les yaourts à boire (ESSAI 10 et ESSAI 50) ont été préparés selon la recette figurant dans le tableau ci-dessous, et contiennent la poudre granulée de ladite invention aux deux taux de substitution différents. Ils ont ensuite été comparés au yaourt ne contenant que du lait ainsi qu'aux yaourts à boire témoin (TEMOIN 10 et TEMOIN 50) préparés en parallèle, dans les mêmes conditions et ne contenant pas la poudre granulée selon la présente invention mais le simple mélange physique des deux constituants.

Les différents yaourts à boire ont été dégustés en aveugle par un jury entrainé et expert en analyse sensoriel de 20 personnes. Les paramètres suivants ont été testés et notés sur une échelle de 1 à 5, 1 étant la note la plus mauvaise et 5 la meilleure : couleur, odeur, goût, onctuosité en bouche, consistance, note générale.

1. Recettes utilisées

|  |  | Poudre granulée | | Mélange « simple » | |
|---|---|---|---|---|---|
| **Taux de substitution** |  | 10% | 50% | 10% | 50% |
|  | TEMOIN | E10 | E50 | T10 | T50 |
| **lait écrémé du commerce** | 86 | 77,5 | 43 | 77,5 | 43 |
| **Poudre granulée selon l'invention en poudre** | / | 8,5 | 43 | 8,5 | 43 |
| **SweetPearl™ P200** | 10 | 10 | 10 | 10 | 10 |

(suite)

| | | | Poudre granulée | | Mélange « simple » | |
|---|---|---|---|---|---|---|
| **Taux de substitution** | | | 10% | 50% | 10% | 50% |
| | | **TEMOIN** | **E10** | **E50** | **T10** | **T50** |
| **NUTRIOSE® FB06** | | 4 | 4 | 4 | 4 | 4 |
| **Ferment probiotique BMY-1** | | qsp | qsp | qsp | qsp | qsp |
| **Total** | | 100 | 100 | 100 | 100 | 100 |

Le SweetPearl™ P200 est le nom commercial du maltitol de la société Demanderesse : il s'agit d'un glucide sous forme de poudre cristalline, issu de l'amidon de blé et de maïs.

Le NUTRIOSE® FB06 est une fibre soluble commercialisée également par la société Demanderesse.

Le ferment utilisé est commercialisé par la société CHR hansen A/S (Danemark).

2. Mode opératoire

- Le NUTRIOSE® FB06 a été dissous dans le lait.

- Le mélange a ensuite été pasteurisé à 90°C pendant 10 minutes.

- Ce mélange pasteurisé a ensuite été homogénéisé grâce à un homogénéisateur NIRO® Soavi (groupe GEA), à une pression de 180 bars.

- L'émulsion ainsi obtenue a ensuite été refroidie à 43°C et maintenue à cette température.

- Les ferments prébiotiques ont été rajoutés au mélange refroidi, et la fermentation a été contrôlée en mesurant continuellement le pH à l'aide d'un pH-mètre.

- La fermentation a été stoppée lorsque le pH du mélange a atteint la valeur de 4,5.

- Le SweetPearl a ensuite été rajouté et le mélange a été homogénéisé avec un homogénéisateur de type ALM2, commercialisé par la société PIERRE GUERIN TECHNOLOGIES (France).

- L'ensemble a été pasteurisé à 90°C pendant 15 secondes, afin d'éliminer les risques de contamination microbiologique.

- Le tout a été refroidi à 5°C avant dégustation.

3. Résultats

| | | Poudre granulée | | Mélange « simple » | |
|---|---|---|---|---|---|
| | | 10% | 50% | 10% | 50% |
| | **TEMOIN** | **E10** | **E50** | **T10** | **T50** |
| **Couleur** | 5 | 4 | 4 | 3 | 4 |
| **Goût** | 5 | 5 | 4 | 3 | 2 |
| **Onctuosité en bouche** | 5 | 5 | 4 | 2 | 2 |
| **Consistance** | 5 | 5 | 5 | 2 | 1 |

(suite)

| | | Poudre granulée | | Mélange « simple » | |
|---|---|---|---|---|---|
| | | 10% | 50% | 10% | 50% |
| | TEMOIN | E10 | E50 | T10 | T50 |
| Note générale | 5 | 5 | 4 | 2 | 2 |

Cet essai démontre parfaitement qu'il est tout à fait possible de substituer une partie des protéines laitières dans un yaourt à boire par la poudre granulée de la présente invention, ou susceptible d'être obtenue selon la mise en oeuvre du procédé de préparation de poudre granulée selon l'invention tel que décrit ci-dessus.

Les yaourts à boire contenant ladite poudre granulée ont été jugés très satisfaisants et identiques au yaourt à boire témoin ne contenant que du lait, avec une très légère préférence mais non significative pour le yaourt à boire E10 (substitution des protéines de lait à un taux de 10%). Les deux yaourts à boire contenant les simples mélanges physiques des deux constituants ont été jugés négativement et leur évaluation démontre parfaitement qu'ils ne se rapprochent absolument pas du yaourt témoin, aussi bien au niveau du goût, de leur onctuosité en bouche et de leur consistance (jugés beaucoup trop liquides). Ainsi, ladite poudre possède des propriétés fonctionnelles gélifiantes qui lui ont été conférées notamment par son procédé de préparation, propriétés qui ne sont pas retrouvées sur le mélange simple des constituants.

## Revendications

1.  Poudre granulée comprenant au moins une protéine végétale et au moins un hydrolysat d'amidon **caractérisée en ce qu'**elle présente

     - un diamètre moyen volumique laser D4,3 compris entre $10\mu m$ et $500\mu m$, de préférence entre $50\mu m$ et $350\mu m$ et encore plus préférentiellement entre $70\mu m$ et $250\mu m$, et
     - une matière sèche, déterminée après étuvage à 130°C pendant 2 heures, supérieure à 80%, de préférence supérieure à 85%, et encore plus préférentiellement supérieure à 90%

    dans laquelle ladite protéine végétale est une protéine de pois, ledit hydrolysat d'amidon est une maltodextrine dont le DE est compris entre 15 et 19,
    la somme des quantités de protéine végétale et d'hydrolysat d'amidon est comprise entre 50 et 100% de la masse totale de ladite poudre granulée (sec/sec), et
    dans laquelle le ratio pondéral de la protéine végétale à l'hydrolysat d'amidon est compris entre 80 :20 et 45 :55,
    dans laquelle les protéines végétales présentent plus de 50 % de protéines de plus de 1.000 Da.

2.  Poudre granulée selon la revendication 1 **caractérisée en ce que** le ratio pondéral de la protéine végétale à l'hydrolysat d'amidon est compris entre 80 :20 et 55 :45.

3.  Poudre granulée selon l'une quelconque des revendications 1-2, **caractérisée en ce qu'**elle présente :

     - une masse volumique apparente comprise entre 0,30 et 0,90 g/ml, de préférence comprise entre 0,40 et 0,60 g/ml ; et
     - une mouillabilité de moins de 60s, de préférence de moins de 30s et plus préférentiellement encore de moins de 10s ; et
     - une absence totale de décantation ; et
     - une capacité émulsifiante supérieure à 50%, de préférence supérieure à 55%, et plus préférentiellement encore supérieure à 60%.

4.  Procédé de fabrication d'une poudre granulée selon l'une quelconque des 1-3 précédentes **caractérisé en ce qu'**il consiste à sécher conjointement au moins deux constituants, et qu'il comprend une étape de mise en contact intime d'au moins une protéine végétale avec au moins un hydrolysat d'amidon, ces étape de mise en contact intime pouvant être menées selon toute méthode connue de l'homme du métier, et notamment selon une technique choisie parmi l'atomisation, la granulation et l'extrusion, et toute combinaison d'au moins deux de ces techniques, telle que ladite étape de mise en contact intime conduise à une matière sèche finale déterminée après étuvage à 130°C pendant 2 heures, supérieure à 80%, de préférence supérieure à 85%, et encore plus préférentiellement supérieure à 90%, dans lequel ladite protéine végétale est une protéine de pois, ledit hydrolysat d'amidon est une maltodextrine dont le DE est compris entre 15 et 19, et le ratio pondéral de la protéine végétale à l'hydrolysat d'amidon est compris entre 80 :20 et 45 :55.

5. Procédé de fabrication d'une poudre granulée selon la revendication 4, **caractérisé en ce que** le procédé comprend une étape d'atomisation d'une suspension d'au moins une protéine végétale et d'au moins un hydrolysat d'amidon, ladite étape d'atomisation étant suivie par une étape de granulation de la poudre atomisée.

6. Procédé de fabrication d'une poudre granulée selon la revendication 4, **caractérisé en ce qu'**il comprend les étapes suivantes :

   1) préparer à une température comprise entre 15 et 70°C, et de préférence entre 15 et 50°C une suspension de protéines de pois et d'hydrolysats d'amidon, dans laquelle :

   - lesdites protéines de pois possèdent une teneur en protéines solubles comprise entre 20 et 99 %, de préférence entre 45 et 90%, plus préférentiellement encore entre 50 et 80%, et en particulier entre 55 et 75% ;
   - lesdits hydrolysats d'amidon étant choisis dans le groupe constitué par les maltodextrines dont le DE est compris entre 15 et 19 et les sirops de glucose dont le DE n'excède pas 47, et de préférence 35, et leurs mélanges quelconques ;
   - le ratio pondéral des protéines de pois aux hydrolysats d'amidon est compris entre 80 :20 et 45 :55 ;
   - la matière sèche de la suspension est comprise entre 25 et 50%, de préférence entre 30 et 40%,

      1') réaliser une première étape optionnelle de traitement thermique à température élevée et pendant un temps court afin de réduire les risques bactériologiques de la suspension obtenue selon 1, ledit traitement pouvant être choisi parmi les traitements HTST (High Temperature Short Time), UHT ;
      1") réaliser une seconde étape optionnelle, d'homogénéisation à haute pression de la suspension obtenue selon 1), et indépendamment de la première étape optionnelle ;

   2) maintenir ou ramener en cas de réalisation de l'étape 1') ladite suspension de protéines de pois et d'hydrolysats d'amidon à une température comprise entre 15 et 80°C, et de préférence entre 15 et 50°C,
   3) atomiser ladite suspension dans une tour d'atomisation équipée d'une buse d'atomisation haute pression avec recyclage des fines particules en tête de tour,
   4) granuler dans ladite tour d'atomisation,
   5) récupérer la poudre granulée ainsi obtenue et comprenant les protéines de pois et les hydrolysats d'amidon.

7. Utilisation de la poudre granulée selon l'une quelconque des revendications 1 à 3 ou susceptible d'être obtenue selon la mise en oeuvre du procédé selon l'une quelconque des revendications 4 à 6 dans la fabrication des émulsions huile/eau, et de préférence dans la fabrication des blanchisseurs de café ou de thé.

8. Utilisation de la poudre granulée selon l'une quelconque des revendications 1 à 3 ou susceptible d'être obtenue selon la mise en oeuvre du procédé selon l'une quelconque des revendications 4 à 6 dans les domaines de la cosmétique, de la détergence, de l'agrochimie, des formulations industrielles, pharmaceutiques, des matériaux de construction, des fluides de forage, en fermentation, nutrition animale et dans des applications alimentaires, de préférence comme agent émulsifiant, foisonnant, stabilisant, épaississant et/ou gélifiant, notamment en substitution de tout ou partie des protéines animales.

9. Emulsion huile/eau, et de préférence blanchisseurs de café ou de thé comprenant la poudre granulée selon l'une quelconque des revendications 1 à 3 ou susceptible d'être obtenue selon la mise en oeuvre du procédé selon l'une quelconque des revendications 4 à 6.

10. Composition cosmétique, détergente, agrochimique, formulations industrielles, pharmaceutiques, matériaux de construction, fluides de forage, milieu de fermentation, composition nutritionnelle animale, applications alimentaires comprenant la poudre granulée selon l'une quelconque des revendications 1 à 3 ou susceptible d'être obtenue selon la mise en oeuvre du procédé selon l'une quelconque des revendications 4 à 6.

11. Agent émulsifiant, foisonnant, stabilisant, épaississant et/ou gélifiant, pouvant être utilisé en substitution de tout ou partie des protéines animales comprenant la poudre granulée selon l'une quelconque des revendications 1 à 3 ou susceptible d'être obtenue selon la mise en oeuvre du procédé selon l'une quelconque des revendications 4 à 6.

12. Formulation alimentaire choisie dans le groupe constitué par les boissons, les produits laitiers, choisi de préférence dans le groupe constitué par les fromages frais et affinés, les fromages fondus éventuellement tartinables, les laits fermentés, les smoothies au lait, les yaourts, les spécialités laitières, les glaces fabriquées à partir de lait, les préparations destinées à la nutrition clinique et/ou à des individus souffrant de dénutrition, les préparations destinées à la nutrition infantile, les mélanges de poudres destinées à des produits de régime ou pour sportifs, les soupes, sauces, et aides culinaires, les produits à base de viande, les produits à base de poissons, tous les types de confiseries, les produits céréaliers, les produits végétariens et plats cuisinés comprenant un agent émulsifiant, stabilisant, épaississant et/ou gélifiant, pouvant être utilisé en substitution de tout ou partie des protéines animales selon la revendication 11.

**Patentansprüche**

1. Granuliertes Pulver, umfassend wenigstens ein pflanzliches Protein und wenigstens ein Stärkehydrolysat, **dadurch gekennzeichnet, dass** es aufweist:

   - einen mittleren Laser-Volumendurchmesser D4,3 zwischen 10 $\mu$m und 500 $\mu$m, bevorzugt zwischen 50 $\mu$m und 350 $\mu$m und noch bevorzugter zwischen 70 $\mu$m und 250 $\mu$m, und
   - eine Trockenmasse, bestimmt nach 2 Stunden Trocknen bei 130°C, von mehr als 80%, bevorzugt von mehr als 85% und noch bevorzugter von mehr als 90%;

   wobei besagtes pflanzliches Protein ein Erbsenprotein ist, besagtes Stärkehydrolysat ein Maltodextrin ist, dessen Dextrose-Äquivalent (DE) zwischen 15 und 19 beträgt,
   die Summe der Mengen an pflanzlichem Protein und Stärkehydrolysat zwischen 50 und 100% des Gesamtgewichts besagten granulierten Pulvers (trocken/trocken) beträgt, und
   wobei das Gewichtsverhältnis von pflanzlichem Protein zu Stärkehydrolysat zwischen 80:20 und 45:55 beträgt,
   wobei von den pflanzlichen Proteinen mehr als 50% der Proteine mehr als 1.000 Da aufweisen.

2. Granuliertes Pulver gemäß Anspruch 1, **dadurch gekennzeichnet, dass** das Gewichtsverhältnis von pflanzlichem Protein zu Stärkehydrolysat zwischen 80:20 und 55:45 beträgt.

3. Granuliertes Pulver gemäß einem der Ansprüche 1 bis 2, **dadurch gekennzeichnet, dass** es aufweist:

   - eine Rohdichte zwischen 0,30 und 0,90 g/ml, bevorzugt zwischen 0,40 und 0,60 g/ml; und
   - eine Benetzbarkeit von weniger als 60 s, bevorzugt von weniger als 30 s und noch bevorzugter von weniger als 10 s; und
   - und ein vollständig ausbleibendes Absetzen; und
   - ein Emulgiervermögen von größer als 50%, bevorzugt von größer als 55% und noch bevorzugter von größer als 60%.

4. Verfahren zur Herstellung eines granulierten Pulvers gemäß einem der vorhergehenden Ansprüche 1 bis 3, **dadurch gekennzeichnet, dass** es aus gemeinsamen Trocknen wenigstens zweier Bestandteile besteht und dass es einen Schritt des innigen Inkontaktbringens wenigstens eines pflanzlichen Proteins mit wenigstens einem Stärkehydrolysat umfasst, wobei diese Schritte des innigen Inkontaktbringens gemäß einem beliebigen dem Fachmann bekannten Verfahren und insbesondere gemäß einer Technik, ausgewählt aus Sprühtrocknen, Granulation und Extrusion und einer beliebigen Kombination aus wenigstens zwei dieser Techniken, durchgeführt werden können, wobei besagter Schritt des innigen Inkontaktbringens zu einer finalen Trockenmasse, bestimmt nach 2 Stunden Trocknen bei 130°C, von mehr als 80%, bevorzugt von mehr als 85% und noch bevorzugter von mehr als 90% führt, wobei besagtes pflanzliches Protein ein Erbsenprotein ist, besagtes Stärkehydrolysat ein Maltodextrin ist, dessen DE zwischen 15 und 19 beträgt, und das Gewichtsverhältnis von pflanzlichem Protein zu Stärkehydrolysat zwischen 80:20 und 45:55 beträgt.

5. Verfahren zur Herstellung eines granulierten Pulvers gemäß Anspruch 4, **dadurch gekennzeichnet, dass** das Verfahren einen Schritt der Sprühtrocknung einer Suspension von wenigstens einem pflanzlichen Protein und wenigstens einem Stärkehydrolysat umfasst, wobei besagtem Sprühtrocknungsschritt ein Schritt der Granulation des sprühgetrockneten Pulvers folgt.

6. Verfahren zur Herstellung eines granulierten Pulvers gemäß Anspruch 4, **dadurch gekennzeichnet, dass** es die folgenden Schritte umfasst:

1) Herstellen einer Suspension von Erbsenproteinen und Stärkehydrolysaten bei einer Temperatur zwischen 15 und 70°C und bevorzugt zwischen 15 und 50°C, wobei:

- besagte Erbsenproteine einen Gehalt an löslichen Proteinen zwischen 20 und 99%, bevorzugt zwischen 45 und 90%, bevorzugter zwischen 50 und 80% und insbesondere zwischen 55 und 75%, besitzen;
- besagte Stärkehydrolysate aus der Gruppe ausgewählt sind, bestehend aus Maltodextrinen, deren DE zwischen 15 und 19 beträgt, und Glucosesirupen, deren DE nicht über 47 liegt, und bevorzugt nicht über 35 liegt, und beliebigen Gemischen davon;
- das Gewichtsverhältnis von Erbsenproteinen zu Stärkehydrolysaten zwischen 80:20 und 45:55 beträgt;
- die Trockenmasse der Suspension zwischen 25 und 50%, bevorzugt zwischen 30 und 40%, beträgt;

1') Durchführen eines optionalen ersten Schrittes der kurzzeitigen thermischen Behandlung bei einer erhöhten Temperatur, um bakterielle Risiken der gemäß 1) erhaltenen Suspension zu verringern, wobei besagte Behandlung aus UHT-, HTST-(High Temperature Short Time)-Behandlungen ausgewählt sein kann;
1") Durchführen eines optionalen zweiten Schrittes der Hochdruck-Homogenisation der gemäß 1) erhaltenen Suspension und unabhängig vom optionalen ersten Schritt;

2) Aufrechthalten oder, im Falle der Durchführung von Schritt 1'), Einstellen der Temperatur besagter Suspension von Erbsenproteinen und Stärkehydrolysaten zwischen 15 und 80°C und bevorzugt zwischen 15 und 50°C,
3) Sprühtrocknen besagter Suspension in einem Sprühturm, der mit einer Hochdrucksprühdüse mit Rückführung feiner Partikel im oberen Teil des Turms ausgestattet ist,
4) Granulieren in besagtem Sprühturm,
5) Wiedergewinnen des auf diese Weise erhaltenen granulierten Pulvers, das die Erbsenproteine und die Stärkehydrolysate umfasst.

7. Verwendung des granulierten Pulvers gemäß einem der Ansprüche 1 bis 3 oder erhältlich gemäß der Anwendung des Verfahrens gemäß einem der Ansprüche 4 bis 6 in der Herstellung von Öl/Wasser-Emulsionen und bevorzugt in der Herstellung von Kaffee- oder Teeweißern.

8. Verwendung des granulierten Pulvers gemäß einem der Ansprüche 1 bis 3 oder erhältlich gemäß der Anwendung des Verfahrens gemäß einem der Ansprüche 4 bis 6 auf den Gebieten der Kosmetik, der Detergenzien, der Agrochemie, in industriellen, pharmazeutischen Formulierungen, Konstruktionsmaterialien, Bohrflüssigkeiten, in der Fermentation, Tierernährung und in Lebensmittelanwendungen, bevorzugt als Emulgator, Quellmittel, Stabilisator, Verdickungsmittel und/oder Gelbildner, insbesondere als Ersatz der gesamten oder eines Teils der tierischen Proteine.

9. Öl/Wasser-Emulsion und bevorzugt Kaffee- oder Teeweißer, umfassend das granulierte Pulver gemäß einem der Ansprüche 1 bis 3 oder erhältlich gemäß der Anwendung des Verfahrens gemäß einem der Ansprüche 4 bis 6.

10. Kosmetische, detergenshaltige, agrochemische Zusammensetzung, industrielle, pharmazeutische Formulierungen, Konstruktionsmaterialien, Bohrflüssigkeiten, Fermentationsmedium, Tiernahrungsmittelzusammensetzung, Lebensmittelanwendungen, umfassend das granulierte Pulver gemäß einem der Ansprüche 1 bis 3 oder erhältlich gemäß der Anwendung des Verfahrens gemäß einem der Ansprüche 4 bis 6.

11. Emulgator, Quellmittel, Stabilisator, Verdickungsmittel und/oder Gelbildner, der/das als Ersatz der gesamten oder eines Teils der tierischen Proteine verwendet werden kann, umfassend das granulierte Pulver gemäß einem der Ansprüche 1 bis 3 oder erhältlich gemäß der Anwendung des Verfahrens gemäß einem der Ansprüche 4 bis 6.

12. Lebensmittelformulierung, ausgewählt aus der Gruppe, bestehend aus Getränken, Milchprodukten, bevorzugt ausgewählt aus der Gruppe, bestehend aus frischen und gereiften Käsen, Schmelzkäsen, eventuell Streichkäsen, fermentierten Milchprodukten, Milch-Smoothies, Joghurts, Milchspezialitäten, aus Milch hergestellten Eisprodukten, Präparaten für die klinische Ernährung und/oder für Personen, die an Mangelernährung leiden, Präparaten für die Säuglingsernährung, Pulvermischungen für Diätprodukte oder für Sportler, Suppen, Saucen und Kochhilfen, Produkten auf Fleischbasis, Produkten auf Fischbasis, allen Arten von Süßwaren, Getreideprodukten, vegetarischen Produkten und Fertiggerichten, umfassend einen Emulgator, einen Stabilisator, ein Verdickungsmittel und/oder einen Gelbildner, der/das als Ersatz der gesamten oder eines Teils der tierischen Proteine gemäß Anspruch 11 verwendet werden kann.

**Claims**

1. A granulated powder comprising at least one vegetable protein and at least one starch hydrolyzate, **characterized in that** it has:

   - a laser volume average diameter D4,3 of between 10 μm and 500 μm, preferably between 50 μm and 350 μm, and even more preferably between 70 μm and 250 μm, and
   - a dry matter content, determined after stoving at 130°C for 2 hours, of greater than 80%, preferably greater than 85%, and even more preferably greater than 90%;

   wherein said vegetable protein is a pea protein, said starch hydrolysate is a maltodextrin of which the DE is between 15 and 19,
   the sum of the amounts of vegetable protein and of starch hydrolyzate is between 50% and 100%, of the total mass of said granulated powder (dry/dry), and
   wherein the weight ratio of the vegetable protein to the starch hydrolyzate is between 80:20 and 45:55,
   wherein the vegetable proteins have more than 50 % of proteins of more than 1,000 Da.

2. The granulated powder as claimed in claim 1, **characterized in that** the weight ratio of the vegetable protein to the starch hydrolyzate is between 80:20 and 55:45.

3. The granulated powder as claimed in any one of claims 1-2, **characterized in that** it exhibits:

   - an apparent density of between 0.30 and 0.90 g/ml, preferably between 0.40 and 0.60 g/ml; and
   - a wettability of less than 60 s, preferably less than 30 s, and even more preferably less than 10 s; and
   - a total absence of settling out; and
   - an emulsifying capacity of greater than 50%, preferably greater than 55%, and even more preferably greater than 60%.

4. A process for manufacturing a granulated powder according to any one of claims 1-3, **characterized in that** it consists in conjointly drying at least two constituents, and **in that** it comprises a step of bringing at least one vegetable protein into intimate contact with at least one starch hydrolyzate, these steps of bringing into intimate contact to be carried out according to any process known to those skilled in the art, and in particular according to a technique chosen from spray-drying, granulation and extrusion, and any combination of at least two of these techniques, such that said step of bringing into intimate contact results in a final dry matter content, determined after stoving at 130°C for 2 hours, of greater than 80%, preferably greater than 85%, and even more preferably greater than 90%, wherein said vegetable protein is a pea protein, said starch hydrolysate is a maltodextrin of which the DE is between 15 and 19, and the weight ratio of the vegetable protein to the starch hydrolyzate is between 80:20 and 45:55.

5. The process for manufacturing a granulated powder according to claim 4, **characterized in that** the process comprises a step of spray-drying a suspension of at least one vegetable protein and of at least one starch hydrolyzate, said spray-drying step being followed by a step of granulation of the spray-dried powder.

6. The process for manufacturing a granulated powder according to claim 4, **characterized in that** it comprises the following steps:

   1) preparing, at a temperature between 15 and 70°C, and preferably between 15 and 50°C, a suspension of pea proteins and of starch hydrolyzates, in which:

      - said pea proteins have a soluble protein content of between 20% and 99%, preferably between 45% and 90%, even more preferably between 50% and 80%, and in particular between 55% and 75%;
      - said starch hydrolyzates are chosen from the group consisting of maltodextrins of which the DE is between 15 and 19 and glucose syrups of which the DE does not exceed 47, and preferably 35, and any mixtures thereof;
- the weight ratio of the pea proteins to the starch hydrolyzates is between 80:20 and 45:55;
- the dry matter content of the suspension is between 25% and 50%, preferably between 30% and 40%,

1') carrying out an optional first step of heat treatment at high temperature and for a short period of time in order to reduce the bacteriological risks of the suspension obtained according to 1, it being possible for said treatment to be chosen from HTST (high temperature short time) and UHT treatments; 1") carrying out an optional second step of high-pressure homogenization of the suspension obtained according to 1), and independently of the optional first step;

2) maintaining or, in the event of step 1') being carried out, bringing back said suspension of pea proteins and of starch hydrolyzates at a temperature of between 15 and 80°C, and preferably between 15 and 50°C; 3) spray-drying said suspension in a spray-drying tower equipped with a high-pressure spray-drying nozzle with recycling of the fine particles at the top of the tower, 4) granulating in said spray-drying tower, 5) recovering the resulting granulated powder comprising the pea proteins and the starch hydrolyzates.

7.  Use of the granulated powder according to any one of claims 1 to 3 or capable of being produced according to the implementation of the process according to any one of claims 4 to 6, in the manufacture of oil/water emulsions, and preferably in the manufacture of coffee or tea whiteners.

8.  Use of the granulated powder as claimed in any one of claims 1 to 3 or capable of being produced according to the implementation of the process as claimed in any one of claims 4 to 6, in the fields of cosmetics, detergence, agrochemistry, industrial or pharmaceutical formulations, construction materials, drilling fluids, in fermentation, in animal feed and in food applications, preferably as an emulsifying, overrun, stabilizing, thickening and/or gelling agent, in particular for totally or partially replacing animal proteins.

9.  An oil/water emulsion, and preferably coffee or tea whiteners, comprising the granulated powder according to any one of claims 1 to 3 or capable of being produced according to the implementation of the process as claimed in any one of claims 4 to 6.

10. A cosmetic, detergent or agrochemical composition, industrial or pharmaceutical formulations, construction materials, drilling fluids, a fermentation medium, an animal nutritional composition or food applications comprising the granulated powder according to any one of claims 1 to 3 or capable of being produced according to the implementation of the process as claimed in any one of claims 4 to 6.

11. An emulsifying, overrun, stabilizing, thickening and/or gelling agent, which can be used for totally or partially replacing animal proteins, comprising the granulated powder according to any one of claims 1 to 3 or capable of being produced according to the implementation of the process as claimed in any one of claims 4 to 6.

12. A food formulation chosen from the group constituted of beverages, dairy products, preferably chosen from the group constituted of fromage frais and ripened cheeses, processed cheesed or processed cheese spreads, fermented milks, milk smoothies, yoghurts, dairy specialties and ice creams produced from milk, preparations intended for clinical nutrition and/or for individuals suffering from undernourishment, preparations intended for infant nutrition, mixtures of powders intended for diet products or for sportspersons, soups, sauces and cooking aids, meat-based products, fish-based products, all types of confectionary, cereal products, vegetarian products and ready meals, comprising an emulsifying, stabilizing, thickening and/or gelling agent, which can be used for totally or partially replacing animal proteins, as claimed in claim 11.

EP 2 403 350 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008066308 A
- EP 0522800 A
- EP 0238946 A
- US 6056949 A
- EP 663797 A
- WO 9819652 A
- WO 2005063056 A
- WO 2005063058 A
- WO 2007017572 A
- EP 1558094 A

**Littérature non-brevet citée dans la description**

- **HOOVER R.** Composition, structure, functionality and chemical modification of legume starches : a review. *Can. J. Physiol. Pharmacol.,* 1991, vol. 69, 79-92
- **C-L HEYDLEY et al.** Developing novel pea starches. *Proceedings of the Symposium of the Industrial Biochemistry and Biotechnology Group of the Biochemical Society,* 1996, 77-87
- **BUCKEE.** *Journal of the Institute of Brewing,* 1994, vol. 100, 57-64
- **J. GUEGUEN.** *Proceedings of european congress on plant proteins for human food,* 1983, vol. 3-4, 267-304
- Encyclopedia of Chemical Technology de Kirk-Othmer. 1978, vol. 22
- Encyclopedia of Chemical Technology de Kirk-Othmer. 1978, vol. 22, 499-521